(19) Europäisches Patentamt
European Patent Office
Office européen des brevets

(11) **EP 4 140 426 B1**

(12) **EUROPÄISCHE PATENTSCHRIFT**

(45) Veröffentlichungstag und Bekanntmachung des Hinweises auf die Patenterteilung:
**24.07.2024   Patentblatt 2024/30**

(21) Anmeldenummer: **22192311.3**

(22) Anmeldetag: **26.08.2022**

(51) Internationale Patentklassifikation (IPC):
**A61B 18/12** *(2006.01)*     *A61B 18/00* *(2006.01)*

(52) Gemeinsame Patentklassifikation (CPC):
**A61B 18/1206;** A61B 2018/00607;
A61B 2018/1273; A61B 2018/128

(54) **ELEKTROCHIRURGIE-GENERATOR MIT MULTILEVEL-INVERTER FÜR HF-HOCHSPANNUNG**

ELECTROSURGICAL GENERATOR WITH MULTILEVEL INVERTER FOR HF HIGH VOLTAGE

GÉNÉRATEUR D'ÉLECTROCHIRURGIE POURVU D'INVERSEUR À NIVEAUX MULTIPLES POUR HAUTE TENSION HF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.08.2021   US 202163237397 P**

(43) Veröffentlichungstag der Anmeldung:
**01.03.2023   Patentblatt 2023/09**

(73) Patentinhaber: **Olympus Winter & Ibe GmbH 22045 Hamburg (DE)**

(72) Erfinder:
• **Dijkstra, Jelle**
  **10781 Berlin (DE)**
• **Fähsing, Thomas**
  **12305 Berlin (DE)**
• **Ramin, Daniel**
  **14558 Nuthetal (DE)**
• **Dietrich, Stefan**
  **14469 Potsdam (DE)**
• **Prezewowsky, Thomas**
  **14513 Teltow (DE)**
• **Schiddel, Stefan**
  **14532 Stahnsdorf (DE)**
• **Becker, Dimitri**
  **10243 Berlin (DE)**

(74) Vertreter: **Glawe, Delfs, Moll Partnerschaft mbB von Patent- und Rechtsanwälten Postfach 13 03 91 20103 Hamburg (DE)**

(56) Entgegenhaltungen:
EP-A1- 2 514 380         EP-A2- 2 779 410
DE-B3- 102013 005 277

**Beschreibung**

Die Erfindung betrifft einen Elektrochirurgie-Generator, der dazu ausgebildet ist, eine hochfrequente Wechselspannung an ein elektrochirurgisches Instrument abzugeben. Er umfasst eine Gleichspannungsversorgung und einen Inverter für Hochspannung, der von der Gleichspannungsversorgung gespeist ist und eine hochfrequente Wechselspannung erzeugt, die an einem Ausgang zum Anschluss des elektrochirurgischen Instruments angelegt ist.

In der Elektrochirurgie wird hochfrequenter Wechselstrom verwendet insbesondere zum Schneiden bzw. Durchtrennen von Gewebe sowie zur Entfernung von Körpergewebe im Sinne einer thermischen Resektion (sogenanntes elektrisches Skalpell). Das Funktionsprinzip beruht auf Erwärmung des zu schneidenden Gewebes. Ein Vorteil liegt darin, dass gleichzeitig mit dem Schnitt auch eine Blutungsstillung durch Verschluss der betroffenen Gefäße erfolgen kann (Koagulation). Hierzu werden beträchtliche Leistungen benötigt, und zwar bei Frequenzen von 200 kHz oder höher bis zu 4000 kHz, typischerweise um die 400 kHz. Bei solchen Frequenzen verhält sich das Körpergewebe wie ein ohmscher Widerstand. Der spezifische Widerstand hängt jedoch stark von der Gewebeart ab, so unterscheiden sich die spezifischen Widerstände von Muskeln, Fett oder Knochen stark voneinander, und zwar bis zum Faktor 1000. Dies führt dazu, dass sich im Betrieb die Lastimpedanz des elektrischen Skalpells abhängig von dem zu schneidenden Gewebe schnell und stark ändern kann, bis hin zu einem Nahezu-Kurzschluss. Das stellt besondere und einzigartige Anforderungen an den Elektrochirurgie-Generator und dessen Hochspannungsbereitstellung. Insbesondere ist eine schnelle Spannungsregelung erforderlich, geeignet für hohe Spannungen im Bereich von einigen Kilovolt und hohe Frequenz in einem weiten Bereich von typischerweise zwischen 200 kHz und bis zu 4 MHz.

Je nach Gewebe und der sich daraus ergebenden Impedanz variieren die Ströme zwischen einigen Milliampere und mehreren Ampere, und zwar hochdynamisch innerhalb kürzester Zeit. Die Wellenform der abgegebenen Wechselspannung kann kontinuierlich sinusförmig sein oder kann moduliert sein mit einem Scheitelfaktor von bis zu 10 bei Modulationsfrequenzen von bis zu etwa 20 kHz.

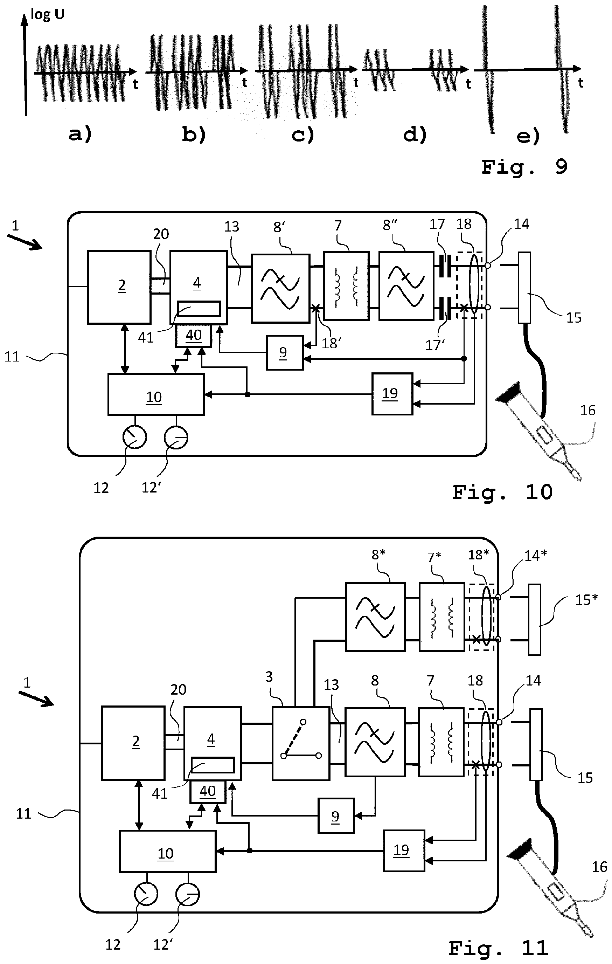

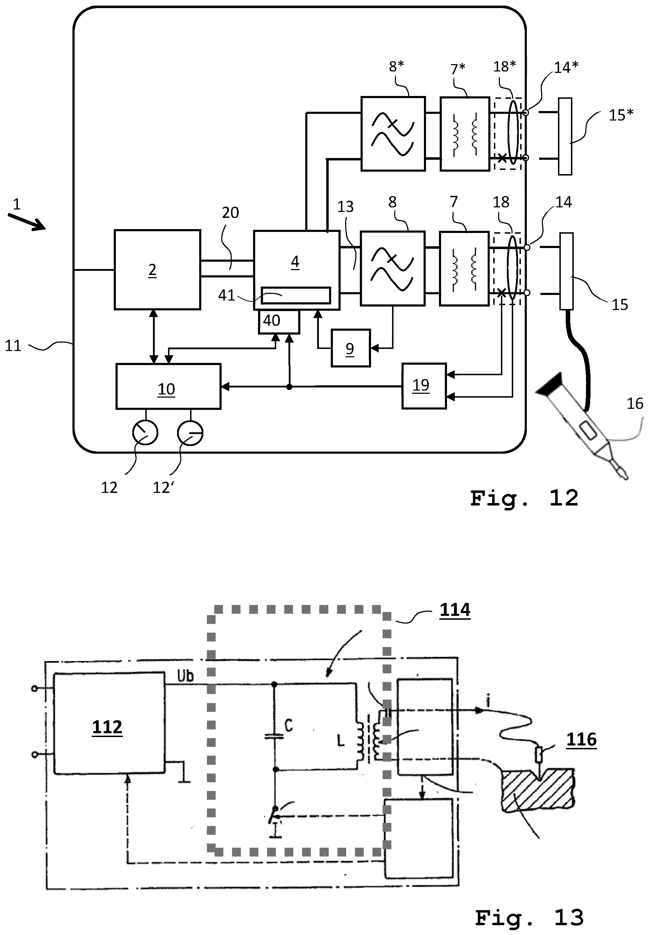

Zur Erfüllung dieser einzigartigen Anforderungen sind Elektrochirurgie-Generatoren typischerweise so aufgebaut, dass sie einen Wechselrichter zur Versorgung des elektrochirurgischen Instruments aufweisen, dem gleichgerichteter Strom aus dem Netz mit unterschiedlicher Spannung zugeführt wird. Der Wechselrichter wiederum ist typischerweise ausgeführt als freischwingender Eintaktgenerator mit einem LC-Resonanzkreis (s. in Fig. 13 zum Stand der Technik den durch punktierte Linie hervorgehobenen Block 114, der von einem Netzteil 112 gespeist ist zur Versorgung eines Instruments 116). Dieser Aufbau ist bewährt (beispielhaft: EP 2 514 380 B1). Allerdings gewinnen in neuerer Zeit immer mehr modulierte Moden an Bedeutung, bei denen die elektrochirurgischen Instrumente getaktet angesteuert werden. Beispiele für solche Moden sind in Fig. 9a bis 9e enthalten. So können die Elektrochirurgie-Generatoren bspw. in einem Schneid-Modus zum Schneiden von Gewebe kontinuierlich eine Spannung von bspw. 600 Volt (Effektivwert) abgeben (Figur 9a) und in einem Koagulations-Modus modulierte Hochspannung mit einer Spitzenspannung von bis zu 4500 V abgeben, aber intervallartig mit kleinem Tastverhältnis (Figur 9e). Verschiedene weitere Moden mit anderen Arten von Spannungs-/Zeitverläufen sind hierbei einstellbar (s. Figur 9b-d). Insbesondere Moden mit kleinem Tastverhältnis und großen, schnellen Spannungssprüngen stellen hohe Anforderungen an die Elektrochirurgie-Generatoren.

Elektrochirurgie-Generatoren mit einem Parallelresonanzkreis ermöglichen zwar die Erzeugung der geforderten hohen Frequenzen, weisen aber eine Reihe von Nachteilen auf. Zum einen ist aufgrund hoher Verluste die Effizienz gering. Zudem treten große Blindströme im Parallelresonanzkreis auf, was größer dimensionierte Komponenten erforderlich macht und zusätzlich den Wirkungsgrad bei niedriger Leistung verschlechtert. Ferner ist die Ausgangsfrequenz lastabhängig, ebenso wie der Scheitelfaktor, was ungünstig ist für stark modulierte Moden. Die Regelung der Ausgangsspannung ist verhältnismäßig langsam, so dass die Anpassung an veränderte Lastimpedanzen nur schlecht ist.

Auf anderen Gebieten, wie bei Audioverstärkern, ist es bekannt als Leistungsstufe Wechselrichter vorzusehen, die nach der Technologie von digitalen Verstärkern, sog. Class-D Verstärkern, aufgebaut sind. Allerdings wird diese Bauart mit ihrer Ausgangsfrequenz im Niederfrequenz-Bereich nicht verwendet für hochfrequente Anwendungen, wie für Elektrochirurgie-Generatoren. Denn die beim Umschalten der Leistungshalbleiter entstehenden Leistungsverluste steigen linear mit der Frequenz und sogar quadratisch mit der Spannung, was bspw. bei etwa sechsfacher Frequenz und ebenfalls sechsfacher Spannung zu einer nicht akzeptablen Steigerung des Verlustleistungsfaktors auf ($6^3=$) 216 führen würde. Dies ist weder im Hinblick auf die Verluste in den Leistungshalbleitern noch unter Wirkungsgradaspekten vertretbar.

Der Erfindung liegt die Aufgabe zugrunde, einen Elektrochirurgie-Generator der eingangs genannten Art im Hinblick auf sein Betriebsverhalten zu verbessern, und zwar insbesondere bei modulierten Moden.

Die erfindungsgemäße Lösung liegt in den Merkmalen des unabhängigen Anspruchs. Vorteilhafte Weiterbildungen sind Gegenstand der abhängigen Ansprüche.

Bei einem Elektrochirurgie-Generator, der dazu ausgebildet ist eine hochfrequente Wechselspannung an ein elektrochirurgisches Instrument abzugeben, umfassend eine Gleichspannungsversorgung und einen Inverter für Hochspannung, der von der Gleichspannungsversorgung gespeist ist und eine hochfrequente Wechselspannung mit variabler Spannung und Frequenz erzeugt, die an einem Ausgang zum Anschluss des elektrochirurgischen Instruments angelegt ist, ist erfindungsgemäß vorgesehen, dass der Inverter als Multilevel-Inverter ausgeführt ist und eine Mehrzahl von kaskadiert geschalteten Wechselrichter-Zellen umfasst, die von einer Steuereinrichtung angesteuert sind.

Kern der Erfindung ist der Gedanke, die typischerweise vom Elektrochirurgie-Generator abzugebende hohe Spannung sowie hohe Frequenz aufzuteilen auf mehrere Wechselrichter-Zellen. Dadurch werden die in den Leistungshalbleitern der einzelnen Wechselrichter-Zelle anfallenden Schaltverluste verringert. Dies gilt sowohl in Bezug auf Höhe (durch die aufgeteilte und damit niedrigere Spannung) wie auch in Bezug auf Häufigkeit (durch die verringerte Schaltfrequenz). Da insbesondere die Verlustleistung quadratisch mit der Spannung steigt, kann auf diese Weise dank der Kaskadierung eine überproportionale Entlastung der Wechselrichter-Zellen mit dem erfindungsgemäßen Multilevel-Inverter erreicht werden. So ergibt sich beispielsweise bei einer Anordnung von zehn Wechselrichter-Zellen für jeden der Wechselrichter-Zellen nur noch ein Zehntel der Schaltvorgänge bei $(1/10)^2$ entsprechend einem Hundertstel der Verlustleistung. Aber nicht nur in Bezug auf Spannungs- und Frequenzfestigkeit ergeben sich Vorteile, sondern auch in Bezug auf die Dynamik. Denn mit den Wechselrichter-Zellen kann die abgegebene Wechselspannung schnell auf Änderungen, insbesondere Sprünge der Lastimpedanz, angepasst werden, wobei die Kurvenform nahezu frei wählbar ist. Die abgegebene Wechselspannung kann somit auch stark moduliert werden einschließlich scharfer und hoher Spannungsspitzen, ohne die Wechselrichter-Zellen mit ihren Leistungshalbleitern zu überlasten. So können insbesondere auch stabile Ausgangsspannungen bei stark gepulsten Moden erreicht werden, mit verlässlichem vorgebbarem Scheitelfaktor auch bei kurzen Tastverhältnissen.

Nachfolgend sollen zuerst einige verwendete Begriffe erläutert werden:
Im Gebiet der Elektrochirurgie-Generatoren werden unter "hochfrequent" Frequenzen typischerweise im Bereich von 200 kHz bis 4000 kHz verstanden. Optional kann bei vorteilhaften Ausführungsformen auch der Ultraschallbereich abgedeckt sein. Unter Ultraschall-Bereich wird ein Frequenzbereich zwischen 20 kHz und 200 kHz verstanden.

Unter "Hochspannung" werden typischerweise Spannungen bis 10kV, vorzugsweise bis 5000 V verstanden.

Die von dem Elektrochirurgie-Generator bereitgestellte Leistung liegt typischerweise im Bereich zwischen 1 und 500 Watt, wobei die Lastimpedanz stark variieren kann und entsprechend sich Ausgangsspannung und Leistungsabgabe ebenso stark und rasch ändern können.

Multilevel-Inverter sind solche eine Wechselspannung aus Gleichspannung erzeugende Inverter, die mehr als zwei von Null verschiedene Spannungslevel an ihrem Ausgang erzeugen können.

Zweckmäßigerweise weisen die Wechselrichter-Zellen ausgangsseitig jeweils eine Potentialentkopplung auf. Hierbei macht sich die Erfindung zunutze, dass ausgangsseitig der Wechselrichter-Zellen definitionsgemäß Wechselspannung anliegt, so dass mit simplen und kostengünstigen Übertragern auf wenig aufwändige Weise (verglichen mit isolierten Einzel-Gleichspannungsquellen, wie sie eingangsseitig erforderlich wären) eine zuverlässige potenzialmäßige Trennung der von den Wechselrichter-Zellen schließlich abgegebenen Spannungen erreicht werden kann.

Bevorzugt ist es, wenn zur Potentialentkopplung Übertrager ausgangsseitig an den Wechselrichter-Zellen vorgesehen sind. Somit sorgt an jeder Wechselrichter-Zelle der jeweilige Übertrager dafür, dass die schließlich von der jeweiligen Wechselrichter-Zelle abgegebene Ausgangsspannung potentialfrei ist. Mit Vorteil sind an dem Ausgang der jeweiligen Wechselrichter-Zelle die Übertrager mit seiner jeweiligen Primärseite angeschlossen ist, wobei Sekundärseiten der Übertrager verkettet sind zur Summierung von Sekundärspannungen der jeweiligen Übertrager, wobei die summierte Spannung über eine Abgabeleitung zum Ausgang zum Anschluss des elektrochirurgischen Instruments geführt ist.

Insgesamt ergibt sich somit eine Verbesserung des Schaltverhaltens in Kombination mit einer deutlichen Verringerung des Aufwands. Zwar bedeutet die Anordnung der Übertrager am Ausgang der Wechselrichter-Zellen, dass die Abgabe von Gleichspannung nicht mehr möglich ist, aber dies ist - wie die Erfindung weiter erkannt hat - auf dem Gebiet der Elektrochirurgie-Generatoren kein Nachteil, sondern ein Vorteil.

Denn es erhöht sich so zusätzlich die Sicherheit für den Patienten, indem der gesamten Wechselrichter-Anordnung deren intrinsische Fähigkeit zur Abgabe von (für den Patienten gefährlichem) Gleichstrom genommen wird. Die Potentialentkopplung, insbesondere Übertrager, am Ausgang der Wechselrichter-Zellen wirken insoweit als ein weiterer Schutzschild für den Patienten.

Vorzugsweise sind die Übertrager jeweils mit einem Transformator als Vorverstärker zum Hochsetzen der Spannung versehen. Damit kann die von den Wechselrichter-Zellen abgegebene Spannung verstärkt werden, wobei zugleich die in der Abgabeleitung fließenden Ströme verringert werden. Besonders zweckmäßig ist es, wenn der Übertrager mit dem jeweiligen Transformator baulich integriert ausgeführt ist. Dies ermöglicht eine besonders kostengünstige und raumsparende Kombination der beiden Funktionen galvanische Trennung einerseits und Verstärken der Spannung andererseits.

Vorzugsweise sind die Wechselrichter-Zellen gespeist von jeweils einer Gleichspannungsquelle. Dabei können die Gleichspannungsquellen voneinander isoliert oder potentialmäßig voneinander getrennt sein. Zwingend ist dies aber nicht, sondern optional kann vorgesehen sein, dass sie über ein Bezugspotential verknüpft sind. Eine aufwändige potenzialmäßige Isolierung der Gleichspannungsquellen am Eingang der Wechselrichter-Zellen ist somit überflüssig.

Es kann aber auch vorgesehen sein, dass mehrere, mindestens zwei, Gruppen von Wechselrichter-Zellen vorgesehen sind, wobei die Wechselrichter der jeweiligen Gruppe von einer Gleichspannungsquelle gemeinsam versorgt sind. Das Zusammenfassen in Gruppen ermöglicht so eine effiziente Ausnutzung der Gleichspannungsquellen, was den Aufwand verringert. Mit der gemeinsamen Versorgung können aber noch weitere Vorteile realisiert sein, wie nachfolgend erläutert wird. Unter "Gruppe" ist hierbei zu verstehen, dass sie mindestens eine Wechselrichter-Zelle umfasst.

Zweckmäßigerweise sind die Gleichspannungsquellen gemeinsam aus der Gleichspannungsversorgung gespeist. Insbesondere kann die Gleichspannungsversorgung einen Gleichspannungszwischenkreis umfassen, der bspw. von einem Netzteil oder direkt von extern mit DC gespeist ist. Aufwendige Bereitstellung von gesonderten, gar potentialgetrennten Gleichspannungsquellen für die Wechselrichter-Zellen ist so nicht mehr erforderlich. Das vereinfacht nicht nur die Bereitstellung der für den Betrieb der jeweiligen Wechselrichter-Zellen erforderlichen Gleichspannung. Es ermöglicht auch eine erhebliche konstruktive Vereinfachung. Denn bei dem Betrieb eines Multilevel-Inverters kann es zu Zuständen kommen, bei denen sich in mindestens einer der Wechselrichter-Zellen die Richtung des Leistungsflusses umkehrt, also Leistung in die Gleichspannungsquelle zurückgespeist wird. Dies bedingt sog. bidirektionale Gleichspannungsquellen, die aufwendiger sind als herkömmliche. Werden viele Gleichspannungsquellen benötigt, bspw. für jeden Wechselrichter eine, so erhöht sich der Aufwand beträchtlich. Indem die Erfindung es aber ermöglicht, die Gleichspannungsquellen nicht mehr trennen zu müssen, sondern sie zusammenschalten zu können, wird ein eventueller Leistungsrückfluss durch eine der Wechselrichter-Zellen in die Gleichspannungsquelle kompensiert durch eine andere der Wechselrichter-Zellen mit regulärem Leistungsfluss in Vorwärtsrichtung. Dies gilt umso mehr bei einer Zusammenfassung der Wechselrichter zu Gruppen mit mehreren Wechselrichter-Zellen. So kommt es im Ergebnis kaum noch oder gar nicht mehr zu dem störenden Leistungsrückfluss. Aber selbst wenn es zum Leistungsrückfluss kommt, so genügt es anstatt vieler, wie bisher, nun nur eine Gleichspannungsquelle bidirektional auszuführen.

Mit Vorteil sind mehrere, mindestens zwei Gruppen von Wechselrichter-Zellen vorgesehen, wobei die Gruppen mit Gleichspannung in unterschiedlicher Höhe versorgt sind, wobei vorzugsweise die eine Gruppe von den mehreren Gruppen mit einer mindestens doppelt so hohen Gleichspannung versorgt ist als eine andere Gruppe von den mehreren Gruppen. Unter "Gruppe" ist hierbei zu verstehen, dass sie mindestens eine Wechselrichter-Zelle umfasst. Indem eine Gruppe von Wechselrichter-Zellen mit einer anderen Gleichspannung versorgt wird, kann die maximale Anzahl der ausgebbaren Spannungslevel erhöht werden verglichen mit einer gleichen Anzahl von Wechselrichter-Zellen, die alle mit der gleichen Spannung gespeist sind. Auf diese Weise kann eine feinere Abstufung der abgegebenen Wechselspannung erreicht werden. Ferner kann weiter vorgesehen sein, dass die Ansteuerung der Wechselrichter-Zellen so ausgebildet ist, um die Zahl der Schaltvorgänge weiter zu verringern, was wiederum zur Verringerung der Verlustleistung beiträgt. Zweckmäßigerweise sind drei oder mehr Gruppen von Wechselrichter-Zellen vorgesehen, wobei die Höhe von deren versorgenden Gleichspannung jeweils verschieden ist. Mit Vorteil kann vorgesehen sein, dass die verschiedenen versorgenden Gleichspannungen insbesondere einer geometrischen Reihe folgen. Vorzugsweise ist eine Aufteilung der versorgender Gleichspannung im Verhältnis 1:3:9, um so mit drei Gruppen ein Höchstmaß an verschiedenen Level erzielen zu können.

Mit Vorteil ist dabei zur Versorgung mindestens einer der Gruppen mit unterschiedlicher Spannung jeweils mindestens ein insbesondere ratiometrischer Gleichspannungswandler vorgesehen. Damit bleibt auch bei Schwankungen in der absoluten Höhe der Gleichspannung das Verhältnis der versorgenden Gleichspannung konstant. Besonders zweckmäßig ist es, wenn der Gleichspannungswandler bidirektional ist, bspw. 12 Volt DC zu 48 Volt DC wandeln kann oder umgekehrt. Damit ist ein flexibler Einsatz insbesondere in Umgebungen mit DC-Versorgung ermöglicht, bspw. in konventionellen Fahrzeugen mit 12 Volt Bordnetz oder in modernen Fahrzeugen mit leistungsfähigen 48 Volt Bordnetz. Je nachdem führt der bidirektionale Gleichspannungswandler dann eine Aufwärtswandlung von 12 Volt auf 48 Volt oder eine Abwärtswandelung von 48 Volt auf 12 Volt durch.

So gelingt es der Erfindung, mit einer auf den ersten Blick verblüffend einfach erscheinenden Maßnahme auf so einfache wie zweckmäßige Weise die bei Multilevel-Invertern auftretenden Schwierigkeiten in Bezug auf die Gleichspannungsquellen, nämlich deren potenzialmäßige Isolierung und deren Fähigkeit zur Bidirektionalität (also auch zur Leistungsaufnahme), auf einen Schlag zu lösen.

Mit Vorteil sind die Gleichspannungsquellen der Wechselrichterzellen galvanisch gekoppelt. Die galvanische Kopplung ermöglicht eine einfache und insgesamt wenig aufwändige Anschaltung der Gleichspannungsquellen an die Wechselrichter-Zellen. Insbesondere ermöglicht dies auch eine Realisierung des Konzepts, wonach eine einzige Quelle zur Speisung der Vielzahl von Wechselrichter-Zellen vorgesehen ist. Zweckmäßigerweise fungiert hierbei als Gleichspannungsquelle jeweils der Gleichspannungszwischenkreis des Elektrochirurgie-Generators. Dies ermöglicht einen vom Konzept her einfachen und robusten Aufbau.

Besonders zweckmäßig ist es, wenn die Gleichspannungsversorgung als eine Festspannungsversorgung ausgebildet ist, insbesondere einen Gleichspannungszwischenkreis mit einem festen Spannungsniveau umfasst. Optional können daran die insbesondere ratiometrischen Gleichspannungswandler angeschlossen sein. Eine solche Festspannungsversorgung ermöglicht eine wesentliche Vereinfachung gegenüber solchen Bauarten, die eine aufwendige spannungsveränderliche Gleichspannung benötigen und entsprechend einen spannungsveränderlichen Gleichspannungszwischenkreis erfordern. Für die Erfindung genügt eine Versorgung mit einer Gleichspannung fester Höhe, die gesamte weitere Spannungsanpassung für den Hunderte oder Tausende von Volt überstreichenden Ausgangsspannungsbereich übernimmt der erfindungsgemäße Multilevel-Inverter.

Die Gleichspannungsversorgung kann intern oder extern angeordnet sein. Sie kann als Netzteil ausgeführt sein zum Anschluss an ein Versorgungsnetz, insbesondere ein Dreh- oder Wechselstromnetz, oder auch ausgebildet sein für eine direkte Speisung mit Gleichspannung. Letzteres ist insbesondere von Vorteil bei mobilen Anwendungen in Fahrzeugen (Speisung mit 24 Volt DC, bei modernen Fahrzeugen auch 48 Volt DC) oder in anderen Umgebungen, die mit DC versorgt sind (bspw. mit 48 Volt DC).

In Bezug auf die Ausführung der Wechselrichter-Zellen als solche ist die Erfindung nicht auf eine Bauart festgelegt. So kann vorgesehen sein, dass die Wechselrichter-Zellen bspw. auch in der Bauart mit Neutralpunkt-Klemmung oder in der Bauart mit fliegendem Kondensator ausgeführt sind. Zellen mit Neutralpunkt-Klemmung sind verhältnismäßig einfach ausgeführt, insbesondere in ihrer zweckmäßigen Ausführung mit Dioden zur Klemmung. So benötigt ein Drei-Level-Inverter lediglich zwei Dioden. Mehr Level sind möglich, wodurch eine feinere Stufung erreicht werden kann und die Spannungsbelastung für jede Diode verringert wird. Allerdings nimmt die Anzahl der erforderlichen Dioden quadratisch zur Anzahl der Level zu, was aus praktischen Gründen die Anzahl der Level begrenzt. In dieser Hinsicht günstiger sind Wechselrichter-Zellen der Bauart mit fliegendem Kondensator. Sie weisen ähnliche Vorteile wie diejenigen mit Dioden auf, jedoch steigt mit zusätzlichem Level die Anzahl benötigter Kondensatoren weniger stark an. Bevorzugt ist jedoch eine Ausführung der Wechselrichter-Zellen so, dass sie in Reihe geschaltet sind. Insbesondere wird sie jeweils in H-Brückenschaltung ausgeführt. Auf diese Weise kann eine an sich beliebige Anzahl von Wechselrichter-Zellen vorgesehen sein, wodurch die auf jede der Wechselrichter-Zellen entfallende Spannungsbelastung sich antiproportional zur Anzahl der Wechselrichter-Zellen verringert. Das verringert nicht nur Spannungsverluste über die Leistungshalbleiter, sondern auch deren Schaltverluste. Überdies kann bei der kaskadierten Anordnung die Anzahl der Schaltvorgänge je Wechselrichter-Zelle verringert werden, was ebenfalls zur Verringerung der Schaltverluste beiträgt.

Zur Ansteuerung des Multilevel-Inverters mit seinen Wechselrichter-Zellen ist zweckmäßigerweise ein Steuersignalgenerator vorgesehen, der dazu ausgebildet ein Referenzsignal für die Ansteuerung des Multilevel-Inverters zu erzeugen. Damit kann eine präzise Kontrolle über die Art der abgegebenen Wechselspannung erreicht werden, was insbesondere in Bezug auf die Einstellbarkeit verschiedenen Moden ein gewichtiger Vorteil. Zweckmäßigerweise ist das Referenzsignal ein Muster für die von dem Elektrochirurgie-Generator abzugebende Wechselspannung, insbesondere hinsichtlich Amplitude, Frequenz, Kurvenform und/oder Tastverhältnis, wobei vorzugsweise die Kurvenform frei wählbar einstellbar ist. Insbesondere wird so erreicht, dass die Frequenz der von den Wechselrichter-Zellen erzeugten Wechselspannung aufgeprägt werden kann, ggf. auch die Kurvenform. Mit Vorteil steuert der Steuersignalgenerator eine Invertersteuerung an, die dazu ausgebildet ist die Wechselrichter-Zellen so anzusteuern, dass sie eine Ausgangsspannung gemäß dem Referenzsignal erzeugen. Weiter sind insbesondere Amplitude, Kurvenform und/oder Tastverhältnis der erzeugten Wechselspannung entsprechend zu dem Referenzsignal.

Zweckmäßigerweise ist weiter vorgesehen, dass die Wechselrichter-Zellen mit variabler Frequenz angesteuert werden. Auf diese Weise kann durch Veränderung des Referenzsignals schneller direkt auf verschiedene Anforderungen reagiert werden. So kann die Frequenz der von den Wechselrichter-Zellen erzeugten Wechselspannung schnell angepasst werden, je nach den Anforderungen des mit dem elektrochirurgischen Instrument bearbeiteten Gewebes. Auch kann so zwischen verschiedenen Modulationsarten rasch und harmonisch gewechselt werden.

Vorzugsweise ist die Invertersteuerung als eine Hochgeschwindigkeits-Steuerung ausgeführt. Diese ist dazu ausgelegt, Ansteuersignale für die Wechselrichter-Zellen mit einer Frequenz von mindestens 150 MHz zu erzeugen, vorzugsweise 200 MHz. Damit können Verzerrungen des Ausgangssignals minimiert werden. Um die Ansteuersignale mit derart hoher Geschwindigkeit bereitstellen zu können, ist vorzugsweise die Invertersteuerung als ein Field-Programmable-Gate-Array (FPGA) ausgeführt.

An der Abgabeleitung, insbesondere im Bereich des Ausgangsanschluss, des Elektrochirurgie-Generators ist zweckmäßigerweise ein Ausgangsübertrager vorgesehen. Dieser dient als galvanische Trenneinrichtung, um so eine weitere Sicherheit dafür zu schaffen, dass an dem Ausgang zum Anschluss des elektrochirurgischen Instruments die abgegebene Wechselspannung zum Schutz der Nutzer sowie des Patienten potentialfrei ist. Insbesondere kann der Ausgangsübertrager als Ausgangstransformator ausgeführt sein und so als Haupt-Spannungsverstärker fungieren. Vorzugsweise ist zusätzlich sekundärseitig am Ausgangsübertrager ein Serienkondensator am Ausgangsanschluss angeordnet. Dieser wirkt als Gleichstromsperre (Blockkondensator) und verhindert so, dass schädlicher Gleichstrom in das elektrochirurgische Instrument und darüber zum Patienten fließt.

Mit Vorteil ist am Ende der Abgabeleitung ein Tiefpassfilter vorgesehen, das vorzugsweise als ein Filter mindestens zweiter Ordnung, insbesondere als ein LC-Filter ausgeführt ist. Mit dem Tiefpassfilter können die aus der hohen Schaltfrequenz der Wechselrichter-Zellen resultierenden hochfrequenten Störungen beseitigt werden. Das Filter ist zweckmäßigerweise so ausgelegt, dass seine Resonanzspitze im Bereich zwischen der maximalen Frequenz für die abgebende Wechselspannung und der effektiven Schaltfrequenz der Wechselrichter-Zellen liegt. Mit einem Filter zweiter Ordnung kann hierbei eine ausreichende Glättung des Signals am Ausgang des Elektrochirurgie-Generators erreicht werden. Das Tiefpassfilter kann vorzugsweise zweigeteilt (zweistufig) ausgeführt sein, wobei mit Vorteil eine Stufe vor und eine Stufe nach dem Ausgangsübertrager angeordnet ist. Es werden so die Vorteile einer quellennahen Glättung mit denen einer ausgangsnahen und somit abschließenden Glättung kombiniert.

Bei Filtern besteht generell eine Gefahr, dass die Resonanzfrequenz des Filters durch Hochfrequenzanteile im Steuersignal, nichtlineare Entitäten im System oder (sprungartige) Änderungen der Lastimpedanz angeregt wird. Um dies zu vermeiden ist eine Dämpfungseinrichtung vorgesehen, die zweckmäßigerweise als aktive Dämpfungseinrichtung ausgebildet ist. Damit kann eine ausreichende Dämpfung für das Tiefpassfilter erzielt werden, und zwar ohne die mit passiven Dämpfungsmaßnahmen einhergehenden unerwünschten Leistungsverluste des Ausgangssignals. Es sei angemerkt, dass auch ein Bandpassfilter in diesem Sinn als Tiefpassfilter fungieren kann, sofern die obere Grenzfrequenz des Passbands hinreichend hoch ist, um die hochfrequenten Störungen zu beseitigen.

Gemäß einer besonders vorteilhaften Ausführungsform, die gegebenenfalls unabhängigen Schutz verdient, umfasst die aktive Dämpfeinrichtung eine Rückführung, die vorzugsweise mindestens einen Stromsensor am Tiefpassfilter aufweist. Handelt es sich hierbei um ein LC-Filter, so ist der Stromsensor zweckmäßigerweise in Reihe an einem Ausgangsanschluss des Tiefpassfilters angeordnet. Damit kann mittels Messung des tatsächlich durch die Kapazität des LC-Filters fließenden Stroms eine aktive Dämpfung durchgeführt werden. Hierdurch verbessert sich das Impulsverhalten deutlich, da mit einer solchen aktiven Dämpfung das Filter genauer abgestimmt sein kann als bei herkömmlicher passiver Dämpfung. Es versteht sich, dass weitere Größen (Zustandsgrößen) hinzugefügt werden können, womit eine noch feinere Abstimmung des Filters erreicht werden kann. Beispielsweise kann dazu ein zweiter Stromsensor vorgesehen sein, der dazu ausgebildet ist, einen Strom am Ausgang zu bestimmen. Vorzugsweise ist ein Querstromdetektor für die Rückführung vorgesehen, durch den (ggf. parasitärer) Stromfluss im Filter und/oder Übertrager bestimmt werden kann. Besonders zweckmäßig ist es, wenn jeweils ein Sensor vor und nach dem Übertrager angeordnet ist. Auf diese Weise können Stromverluste durch parasitäre Querkapazitäten, insbesondere im Übertrager am Ausgang, erfasst und ggf. kompensiert werden. Unter "Quer-" wird hierbei ein Stromfluss bzw. eine Kapazität zwischen den beiden Wechselspannungsleitern der Abgabeleitung bzw. des Ausgangs des Elektrochirurgie-Generators verstanden.

Vorzugsweise ist die aktive Dämpfungseinrichtung so ausgeführt, dass sie mit einem Ausgangssignal auf den Multilevel-Inverter wirkt, insbesondere eingekoppelt ist in die Ansteuerung der Wechselrichter-Zellen. Hierbei wird der Ansteuerung der Wechselrichter-Zellen ein entsprechendes Korrektursignal überlagert, wodurch die von den Wechselrichter-Zellen abgegebene Leistung entsprechend beeinflusst wird. Somit kann das Ausgangssignal der Dämpfungseinrichtung direkt an der Leistungsquelle wirken, um so das Entstehen ungünstiger Schwingungen und/oder ungünstigen Impulsverhaltens gleich im Ansatz zu bekämpfen. Technisch ist dies mit Vorteil so gelöst, dass das Ausgangssignal der Dämpfungseinrichtung an die Ansteuerung der Wechselrichter-Zellen angelegt ist, und ein Referenzsignal für die Wechselrichteransteuerung modifiziert, woraus dann wiederum entsprechend modifizierte Ansteuersignale für die Stromventile der Wechselrichter-Zellen bestimmt werden. Die Ansteuerung der Wechselrichter-Zellen wird also dynamisch von der aktiven Dämpfungseinrichtung modifiziert. Auf diese Weise wird die Ausgangsspannung des Multilevel-Inverters abhängig von dem Ausgangssignal der Dämpfungseinrichtung gesteuert.

Bei einer weiteren vorteilhaften Ausführungsform der Erfindung ist vorzugsweise ein weiterer Ausgang vorgesehen, und der Multilevel-Inverter ist ferner dazu ausgebildet, eine weitere Wechselspannung zu erzeugen, die an dem weiteren Ausgang angelegt ist. Zweckmäßigerweise weist die weitere Wechselspannung eine niedrigere Frequenz als die hochfrequente Wechselspannung am Ausgang für den Anschluss des elektrochirurgischen Instruments auf. Vorzugsweise liegt diese niedrigere Frequenz im Ultraschallbereich. Damit erweitert sich das Einsatzspektrum des Elektrochirurgie-Generators beträchtlich, da so auch Instrumente der Ultraschallchirurgie angeschlossen und betrieben werden können. Dem Chirurgen eröffnet dies die Möglichkeit, je nach Bedarf mit geringstem Aufwand auf ein Instrument der Ultraschallchirurgie zu wechseln, ohne dass dazu ein ganz anderer Generator bereitgestellt und in Betrieb genommen zu werden braucht. Auch können Instrumente verwendet werden, die gleichzeitig mit Ultraschall und Hochfrequenz betrieben werden.

Mit Vorteil ist mindestens eine Umschalteinrichtung vorgesehen, welche dazu ausgebildet ist den Multilevel-Inverter wahlweise mit einem der Ausgänge zu verbinden. Auf diese Weise ist dem Chirurgen ein schneller und zügiger Wechsel des Ausgangs ermöglicht, und zwar auch intraoperativ. So kann eine optimale Anpassung des chirurgischen Instruments an die patientenindividuellen Anforderungen je nach den konkret am Situs vorgefundenen Bedingungen rasch und einfach erfolgen.

Zweckmäßigerweise sind an dem Multilevel-Inverter die Wechselrichter-Zellen schaltungsmäßig aufgeteilt, wobei mindestens ein Teil der Wechselrichter-Zellen für einen Anschluss an dem mindestens einen weiteren Ausgang vorgesehen ist und ein anderer Teil der Wechselrichter-Zellen weiterhin den (ersten) Ausgang versorgt. Damit ist es ermöglicht, den weiteren Ausgang zeitgleich zu betreiben, so dass im Ergebnis sowohl ein elektrochirurgisches Instrument am (ersten) Ausgang und ein ultraschallchirurgisches Instrument am weiteren Ausgang betrieben werden kann. Die sozusagen eigenständige Wechselrichter-Zelle für den weiteren Ausgang bietet ferner den Vorteil, dass damit - abgesehen von der unterschiedlichen Frequenz der abgegebenen Wechselspannung - eine Anpassung der am weiteren Ausgang abgegebenen weiteren Wechselspannung auf andere Spannungs- oder Stromanforderungen des ultraschallchirurgischen Instruments ermöglicht ist. So können auch ultraschallchirurgische Instrumente mit abweichender Charakteristik, bspw. mit stark unterschiedlichem Innenwiderstand, sicher und zuverlässig angesteuert werden.

Die Erfindung wird nachfolgend näher unter Bezugnahme auf vorteilhafte Ausführungsformen beispielhaft erläutert. Es zeigen:

Fig. 1　　　eine schematische Darstellung eines Elektrochirurgie-Generators gemäß einem Ausführungsbeispiel mit einem angeschlossenen elektrochirurgischen Instrument;

Fig. 2a, b　Blockdiagramme zu Ausführungsbeispielen für einen Multilevel-Inverter des Elektrochirurgie-Generators gemäß Fig. 1 mit kaskadierten Wechselrichter-Zellen;

Fig. 3　　　einen schematisierten Schaltplan von zwei der Wechselrichter-Zellen;

Fig. 4a-c　Diagramme zu Spannungs- und Signalverläufen für Schaltelemente der beiden Wechselrichter-Zelle gemäß Fig. 3;

Fig. 5　　　einen beispielhaften Schaltplan für den Multilevel-Inverter mit mehreren kaskadierten Wechselrichter-Zellen;

Fig. 6a, b　schematisierte Schaltpläne zu alternativen Ausführungen der Wechselrichter-Zelle;

Fig. 7a, b　Spannungsverläufe am Ausgang ohne Rückkopplung bei hochohmiger Last bzw. Kurzschluss;

Fig. 8a, b　Spannungsverläufe am Ausgang mit Rückkopplung bei hochohmiger Last bzw. Kurzschluss;

Fig. 9a-e　Darstellung verschiedener Spannungs-/Zeitverläufe in der Hochfrequenzchirurgie;

Fig. 10　　eine schematische Darstellung eines Elektrochirurgie-Generators gemäß einem anderen Ausführungsbeispiel;

Fig. 11　　eine schematische Darstellung eines Elektrochirurgie-Generators gemäß einem weiteren Ausführungsbeispiel;

Fig. 12　　eine schematische Darstellung einer Variante des weiteren Ausführungsbeispiels gemäß Fig. 11; und

Fig. 13　　einen Schaltplan zu einem Wechselrichter gemäß dem Stand der Technik.

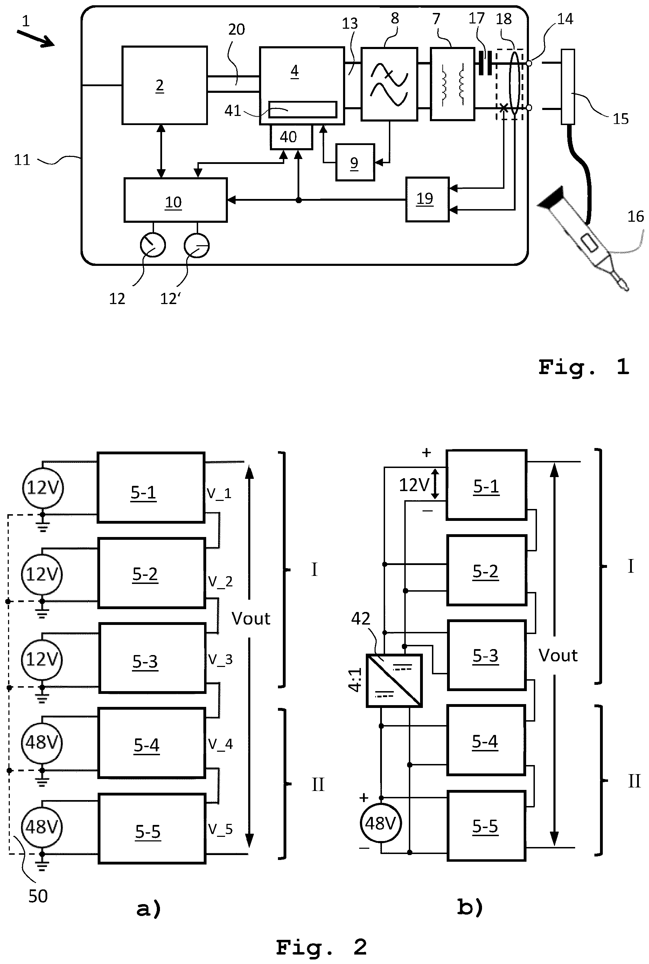

Ein Elektrochirurgie-Generator gemäß einem Ausführungsbeispiel der Erfindung ist in Figur 1 dargestellt. Der in seiner Gesamtheit mit der Bezugsziffer 1 bezeichnete Elektrochirurgie-Generator umfasst ein Gehäuse 11, das mit einem Anschluss 14 für ein elektrochirurgisches Instrument 16 versehen ist, in dem dargestellten Ausführungsbeispiel handelt es sich hierbei um ein elektrisches Skalpell. Es ist über einen Anschlussstecker 15 eines Hochvoltverbindungskabels mit dem Anschluss 14 des Elektrochirurgie-Generators 1 verbunden. Die Leistungsabgabe an das elektrochirurgische Instrument 16 kann über einen Leistungssteller 12 verändert werden.

Zur Leistungsversorgung des Elektrochirurgie-Generators 1 ist eine Gleichspannungsversorgung 2 vorgesehen, welche über ein Netzanschlusskabel (nicht dargestellt) mit dem öffentlichen Stromnetz verbunden werden kann und daraus gespeist ist. Bei der Gleichspannungsversorgung 2 handelt es sich bei dem dargestellten Ausführungsbeispiel um ein Hochspannungsnetzteil (High Voltage Power Supply - HVPS). Es umfasst einen Gleichrichter und speist einen Gleichspannungszwischenkreis 20 mit Gleichspannung, deren Höhe vorzugsweise fest ist und beispielsweise 48 Volt beträgt. Es soll aber nicht ausgeschlossen sein, dass die Gleichspannungshöhe variabel ist zwischen 0 und etwa 400 Volt, wobei die absolute Höhe der Gleichspannung insbesondere abhängen kann von der eingestellten Leistung, der Art des elektrochirurgischen Instruments 16 und/oder dessen Lastimpedanz, die wiederum abhängt von der Art des behandelten Gewebes. Notwendig ist ein internes Netzteil jedoch nicht, so kann die Gleichspannungsversorgung auch durch ein externes Netzteil realisiert sein, oder es ist eine direkte DC-Speisung vorgesehen, bspw. 24 Volt in Fahrzeugen oder 48 Volt bei stationären Anwendungen.

Von dem Gleichspannungszwischenkreis 20 gespeist ist ein Inverter, der aus zugeführter Gleichspannung hochfrequente Wechselspannung im Hochspannungsbereich von einigen Kilovolt erzeugt, mit vorgebbaren Frequenzen im Bereich zwischen 200 kHz und 4 MHz. Der Inverter ist in der Bauart als Multilevel-Inverter 4 ausgeführt, wie nachfolgend noch näher erläutert werden wird. Frequenz und Kurvenform der vom Multilevel-Inverter 4 zu erzeugenden hochfrequenten Wechselspannung sind hierbei vorgegeben von einer Invertersteuerung 41 auf Basis eines von einem Steuersignalgenerator 40 erzeugten Referenzsignals. Die vom Multilevel-Inverter 4 erzeugte hochfrequente Hochspannung wird über einen Tiefpass 8 geführt und einen als Ausgangstransformator zum Hochsetzen der Spannung fungierenden Ausgangsübertrager 7, abgesichert durch einen in Serie angeordneten Blockkondensator 17 gegen unerwünschte Gleichstromanteile, am Anschluss 14 ausgegeben als Uout zum Anschluss für das elektrochirurgische Instrument 16. Ferner werden Spannung und Strom der vom Multilevel-Inverter 4 erzeugten und ausgegebenen Hochspannung gemessen mittels eines kombinierten Spannung- und Stromsensors 18 und die Messsignale werden einer Verarbeitungseinheit 19 zugeführt, welche die entsprechenden Daten über die abgegebene Spannung, Strom und Leistung als Rückführung an den Steuersignalgenerator 40 sowie an eine Betriebssteuerung 10 des Elektrochirurgie-Generators 1 anlegt. An die Betriebssteuerung 10 ist auch der Leistungssteller 12 angeschlossen. Die Betriebssteuerung 10 ist ferner dazu ausgebildet, verschiedene sog. Moden einzustellen, bei denen es sich typischerweise um eingespeicherte Spannungs-/Zeitverläufe handelt. Zur Auswahl des Modus durch den Nutzer ist ein Auswahlschalter 12' vorgesehen. Die Betriebssteuerung 10 wirkt ferner zusammen mit dem Steuersignalgenerator 40, der dazu ausgebildet ist das Referenzsignal für die auszugebende Wechselspannung, insbesondere in Bezug auf Amplitude, Frequenz, Kurvenform und Tastverhältnis zu erzeugen.

Der Multilevel-Inverter 4 umfasst eine Mehrzahl von in Reihe geschalteten Wechselrichter-Zellen 5, die angesteuert sind von einer Invertersteuerung 41. Es wird nun Bezug genommen auf Figur 2a. Bei dem dort dargestellten Ausführungsbeispiel ist an dem Eingang (in der Zeichnung linksseitig dargestellt) jeder der Wechselrichter-Zellen 5 eine Gleichspannungsquelle mit einer bestimmten Gleichspannung angeschlossen. Die jeweilige Wechselrichter-Zelle 5 erzeugt daraus eine Wechselspannung, die an dem Ausgang (in der Zeichnung rechtsseitig dargestellt) der jeweiligen Wechselrichter-Zelle 5 als Wechselspannung abgegeben wird. Die Anzahl der Wechselrichter-Zellen ist nicht beschränkt und an sich beliebig. Die Wechselrichter-Zellen 5 sind in der Figur 2a durchnummeriert mit der Bezeichnung "5-1", "5-2" bis "5-5", wobei die Anzahl 5 beispielhaft ist und eine beliebige Anzahl von mindestens zwei Wechselrichter-Zellen vorgesehen sein kann. Die am Eingang der jeweiligen Wechselrichter-Zelle 5 angelegten Gleichspannungen sind optional potentialmäßig gekoppelt über eine Leitschiene 50. Entsprechend ist die am Ausgang der jeweiligen Wechselrichter-Zelle 5 ausgegebene Wechselspannung bezeichnet als 'V_1", "V_2" bis hin zu "V_5". Durch eine Reihenschaltung der Wechselrichter-Zellen 5 erfolgt eine Addition von deren Ausgangsspannungen, sodass schließlich sich als Gesamt-Ausgangsspannung ergibt:

$$V_{out} = \sum_{i=1}^{N} V\_i$$

Die Anzahl der mit den "N" Wechselrichter-Zellen 5 realisierbaren Spannungsstufen ist hierbei mindestens

$$2N + 1$$

unter der Annahme, dass die am Eingang der Wechselrichter-Zellen 5 angelegten Gleichspannungen "Vin_1", "Vin_2" bis "Vin_N" sämtlich gleichgroß sind. So ergeben sich beispielsweise bei einer Anzahl von fünf Wechselrichter-Zellen 5 insgesamt elf mögliche Spannungsstufen für die Gesamt-Ausgangsspannung Vout.

Die Anzahl der Spannungsstufen kann beträchtlich gesteigert werden bei identischer Anzahl der Wechselrichter-Zellen 5, wenn diese zumindest gruppenweise mit Gleichspannung verschiedener Höhe gespeist sind. Eine derartige Konfiguration ist in Figur 2a, b gezeigt. Dort sind zwei Gruppen von Wechselrichter-Zellen gebildet: eine erste Gruppe I mit drei Wechselrichter-Zellen 5-1, 5-2 bis 5-3, die von einer Gleichspannungsquelle mit niedriger Gleichspannung, im Beispiel 12 V, gespeist sind; und eine zweite Gruppe II mit zwei Wechselrichtern-Zellen 5-4, 5-5, die von einer anderen Gleichspannungsquelle mit höherer Gleichspannung, im Beispiel 48 V, gespeist sind. Die erste Gruppe I enthält Wechselrichter-Zellen mit niedriger Spannung, und die zweite Gruppe II enthält Wechselrichter-Zellen mit hoher Spannung. Zwar erhöht sich hier der Aufwand in Bezug auf Gleichspannungsquellen, weil nunmehr zwei statt einer benötigt werden. Aber dafür erhöht sich die Zahl der Spannungsstufen beträchtlich, und zwar ausgehend von elf mehr als verdoppelt auf 23 Spannungsstufen. Die Anzahl der so erreichbaren Spannungsstufen folgt der Formel

$$2 * (mHVc * r + nLVc) + 1 \quad ,$$

wobei mHVc für die Anzahl der Wechselrichter-Zellen mit höherer Gleichspannung (in obigem Beispiel m=2), nLVc für die Anzahl der Wechselrichter-Zellen mit niedriger Gleichspannung (in obigem Beispiel n=3) und r für das Verhältnis von höherer zu niedriger Gleichspannung (in obigem Beispiel r=4) steht.

Hierbei brauchen die beiden Spannungsquellen potenzialmäßig nicht voneinander isoliert zu sein, sondern sie können sich ein gemeinsames Bezugspotential teilen, wie es in Figur 2a realisiert ist mittels der Leitschiene 50. Dies ermöglicht es ferner aus der höheren Gleichspannung, bei der es sich beispielsweise um die Gleichspannung im Zwischenkreis 20 handeln kann, die niedrigere Gleichspannung mittels eines Gleichspannungswandlers 42, insbesondere eines DC/DC-Tiefsetzstellers, zu erzeugen. Im vorliegenden Beispiel wie in Fig. 2b dargestellt wäre er ausgelegt für eine ratiometrische Versorgung mit einem Untersetzungsverhältnis von 4:1. Ein Vorteil dieser Konfiguration mittels ratiometrischer Versorgung ist, dass Änderungen oder Schwankungen in der höheren Gleichspannung sich dann in proportionaler Form widerspiegeln in der niedrigeren Gleichspannung, so dass die relative Stufung erhalten bleibt. Damit kann beispielsweise eine Erhöhung um der Ausgangsspannung um 12V auf zwei verschiedene Weisen erfolgen, einmal konventionell durch Zuschalten einer weiteren 12V-Wechselrichter-Zelle oder durch Zuschalten einer 48V-Wechselrichter-Zellen kombiniert mit Wegschalten von drei 12V-Wechselrichter-Zellen.

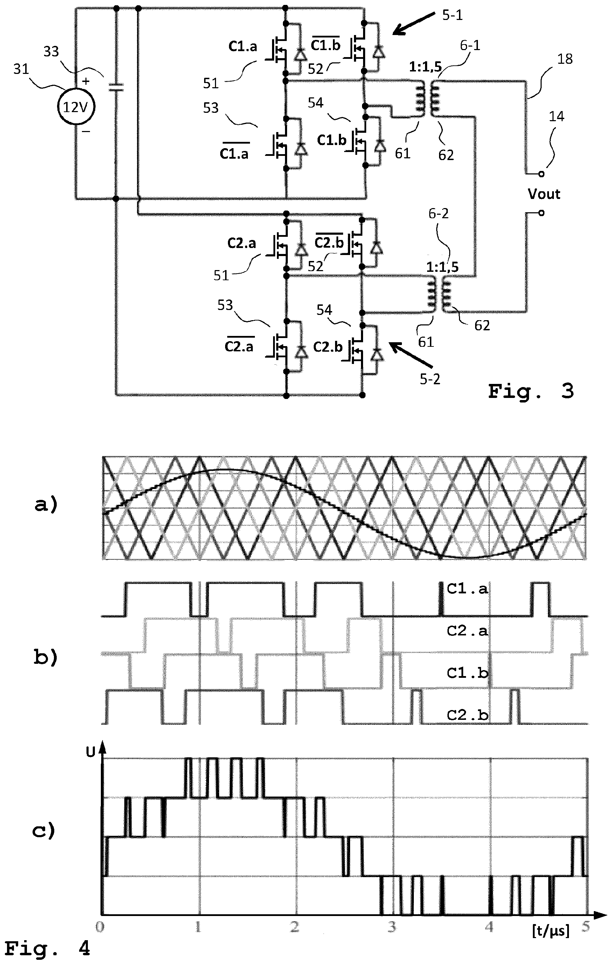

Der Aufbau der einzelnen Wechselrichter-Zellen 5 und deren Zusammenwirken sind in dem schematisierten Schaltplan gemäß Figur 3 beispielhaft dargestellt. Insgesamt sind dort zwei Wechselrichter-Zellen 5-1 und 5-2 in kaskadierter Anordnung gezeigt, um so auch deren gegenseitige Verschaltung darzustellen. Am linken Bildrand ist die gemeinsame Gleichspannungsquelle 31 dargestellt mit einer Versorgungsspannung Vin von 12 Volt. Ihr zugeordnet ist ein Stabilisierungskondensator 33. Sie versorgen die beiden Wechselrichter-Zellen 5-1 und 5-2. Nachfolgend wird zuerst Bezug genommen auf die Schaltung der Wechselrichter-Zelle 5-1. Vorgesehen sind vier Leistungsschalter, die als Stromventile fungieren und in H-Brückenschaltung angeordnet sind. Bei den Leistungsschaltern handelt sich um Halbleiterleistungsschalter, beispielsweise ausgeführt als IGBT, MOS-FET, GaN-FET. Die Leistungsschalter 51, 53 sind in Reihe geschaltet und bilden einen ersten Zweig, und die Leistungshalbleiter 52, 54 sind ebenfalls in Reihe geschaltet und bilden einen zweiten Zweig. Die Mittelpunkte der beiden Zweige sind rausgeführt und an die beiden Enden einer Primärwicklung 61 eines ersten Übertragers 6-1 angeschlossen. Der Übertrager 6-1 weist ferner eine Sekundärwicklung 62 auf und dient zur Potentialtrennung, wobei er optional ferner ein Übersetzungsverhältnis zur Vorverstärkung der Spannung aufweisen kann, im dargestellten Beispiel beträgt es 1:1,5 (es sei angemerkt, dass auch ein anderes Übersetzungsverhältnis vorgesehen sein kann, bspw. mit einem Übersetzungsverhältnis von 1:1, insbesondere wenn keine Vorverstärkung erreicht werden soll). An die Sekundärwicklung 62 angeschlossen ist eine Abgabeleitung 13, die zum Ausgang 14 des Elektrochirurgie-Generators 1 führt (ggf. über ein in Fig. 3 nicht dargestelltes Tiefpassfilter).

Die beiden Leistungsschalter 51, 53 des ersten Zweigs sind mit einem gemeinsamen Signal C1.a angesteuert, wobei dem Leistungsschalter 53 dieses Signal invertiert zugeführt ist. Entsprechend sind die beiden Leistungsschalter 52, 54 des zweiten Zweigs ebenfalls mit einem gemeinsamen Signal C 1.b angesteuert, wobei dem Leistungsschalter 52 dieses Signal invertiert zugeführt ist. Das bedeutet, dass bei einem HIGH-Signal von C1.a der Leistungsschalter 51 durchschaltet und der Leistungsschalter 53 sperrt, also der erste Leistungszweig ein positives Potenzial an den oberen Anschluss der Primärwicklung 61 des Übertragers 6-1 anlegt. Entsprechend schaltet im zweiten Leistungszweig bei einem HIGH-Signal von C2.b der Leistungsschalter 54 durch, während der Leistungsschalter 52 sperrt. Damit legt der zweite Leistungszweig ein negatives Potenzial an den unteren Anschluss der Primärwicklung 61 an. Bei LOW-Signal von C1.a bzw. C1.b gilt dies entsprechend umgekehrt, d. h. die Polarität an der Primärwicklung 61 kehrt sich um. Somit wird eine Wechselspannung durch die Wechselrichter-Zelle 5-1 erzeugt und an der Primärwicklung 61 des Übertragers 6-1 angelegt.

Die zweite Wechselrichter-Zelle 5-2 weist einen identischen Aufbau auf, und ist in gleicher Weise von der Gleichspannungsquelle 31 versorgt wie die erste Wechselrichter-Zelle 5-1. In der Figur sind daher für gleichartige Elemente dieselben Bezugsziffern verwendet. Angesteuert wird sie von den Steuersignalen C2.a und C 2.b in entsprechender Weise wie vorstehend beschrieben. Sie gibt somit an seinem Ausgang ebenfalls eine Wechselspannung aus, die an einer Primärwicklung 61 eines zweiten Übertragers 6-2 angelegt ist. Da die beiden Wechselrichter-Zellen 5-1 und 5-2 von derselben Gleichspannungsquelle 31 gespeist sind, sind sie potenzialmäßig verbunden. Das bedeutet, dass die von den Wechselrichter-Zellen 5-1 und 5-2 unmittelbar ausgegebenen Wechselspannungen nicht ohne weiteres addiert werden können, da sie bezüglich ihres Potenzials miteinander verknüpft sind. Indem diese ausgegebenen Wechselspannung aber jeweils den Übertragern 6-1 und 6-2 zugeführt werden, sind die von den Übertragern 6-1 und 6-2 ausgegebenen Wechselspannungen jeweils potentialfrei und können ohne weiteres miteinander addiert werden zu einer gemeinsamen Ausgangsspannung, die an der Abgabeleitung 13 anliegt.

Das Schaltverhalten der Leistungsschalter 51 bis 54 unter der Wirkung der Steuersignale C1.a, C1.b, C2.a und C2.b, wie sie von der Invertersteuerung 41 beispielsweise mittels der an sich bekannten PWM-Steuerung erzeugt werden, ist in Figur 4 dargestellt. Figur 4a zeigt die Gewinnung der Steuersignale C1.a, C1.b, C2.a und C2.b. Die Invertersteuerung 41 stellt für jedes der Steuersignale ein sägezahnförmiges Trägersignal mit einer Frequenz von 1 MHz bereit, die gleichmäßig um 90° phasenversetzt zueinander sind. Diese vier Trägersignale sind in Figur 4a durch vier versetzte Sägezahn-Züge dargestellt. Ferner dargestellt ist das für die PWM-Modulation benötigte Modulationssignal, vorliegend gebildet durch das Referenzsignal als eine Sinusschwingung mit einer Frequenz von 200 kHz. Die sich durch die Modulation ergebenden Signalfolgen für die vier Steuersignale C1.a, C1.b, C2.a und C2.b, wie sie von der Invertersteuerung 41 ausgegeben werden für die Wechselrichter-Zellen 5-1 und 5-2, sind in Figur 4b dargestellt. Es handelt sich um reine Rechtecksignalfolgen, die jeweils nur einen 1-bit-Schaltzustand kennen. Werden die Leistungsschalter 51 bis 54 der beiden Wechselrichter-Zellen 5-1 und 5-2 in der vorstehenden beschriebenen Weise mit diesen Signalfolgen für die Steuersignale angesteuert, und die von den beiden Wechselrichter-Zellen 5-1 und 5-2 jeweils abgegebenen Spannungen durch die Übertrager 6-1 und 6-2 addiert, dann ergibt sich am Ausgang 14 schließlich der in Figur 4c dargestellte Spannungsverlauf. Aus den vier 1-bit-Steuersignalen wird so eine approximiert sinusförmige Ausgangsspannung erzeugt, die fünf Spannungslevel aufweist.

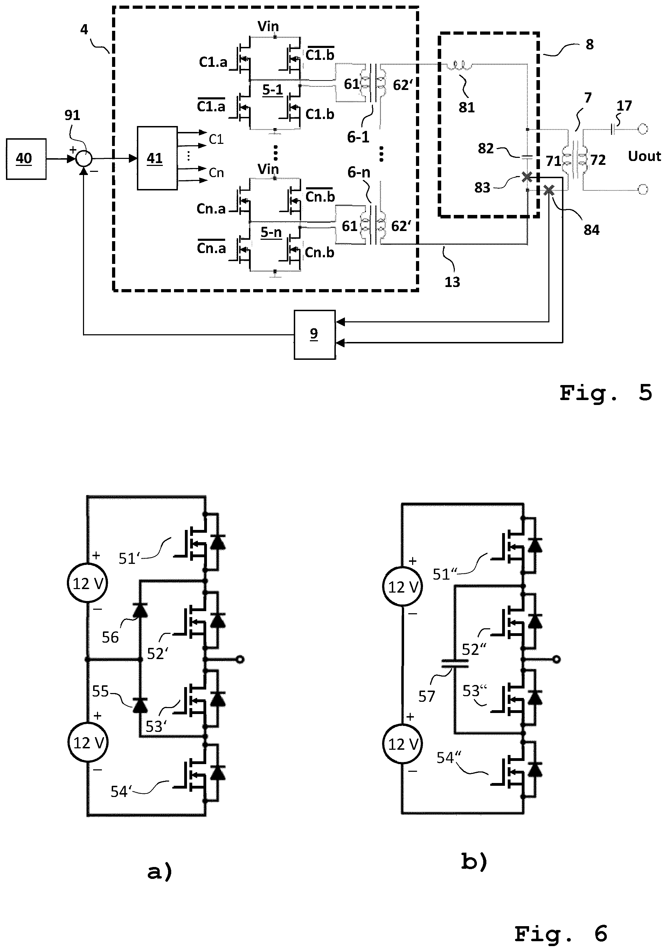

Ein beispielhafter Schaltplan für den Multilevel-Inverter 4 und seine Anbindung an benachbarte Komponenten ist in Figur 5 dargestellt. Man erkennt den Multilevel-Inverter 4 mit seiner Vielzahl von Wechselrichter-Zellen, dargestellt durch die Wechselrichter-Zelle 5-1 bis hin zur Wechselrichter-Zelle 5-n. Sie legen die von ihnen erzeugte Wechselspannung jeweils an Primärwicklungen 61 der ihnen zugeordneten Übertrager 6-1 bis 6-n. Bei diesem Ausführungsbeispiel seien die Übertrager so ausgeführt, dass deren Sekundärwicklungen 62' eine höhere Anzahl von Windungen aufweisen als die Primärwicklung 61. Damit sind sie als kombinierte Übertrager und Transformatoren ausgebildet und sorgen so nicht nur für eine Potentialentkopplung, sondern auch für eine zusätzliche Spannungsverstärkung. Die Sekundärwicklungen 62' sind in Reihe geschaltet, so dass sich deren verstärkte Spannungen summieren zu einer erhöhten Gesamtspannung.

Die Gesamtspannung wird ausgegeben an die Abgabeleitung 13, an deren Ende das Tiefpassfilter 8 angeordnet ist. Es ist als Filter zweiter Ordnung ausgeführt und umfasst eine Induktivität 81 sowie eine dazu in Reihe geschaltete Kapazität 82. Es sei angemerkt, dass auch Streu-Induktivitäten der Übertrager 6-1 bis 6-n zu der Induktivitäten 81 des Tiefpassfilters beitragen und diese ggf. zumindest zum Teil ersetzen können. Das Tiefpassfilter 8 ist so abgestimmt, dass Störungen in der erzeugten Wechselspannung aufgrund der Schaltfrequenz der Leistungsschalter in den Wechselrichter-Zellen des Multilevel-Inverters 4 ausgefiltert werden. Der Ausgang des Tiefpassfilters 8 ist angelegt an eine Primärwicklung 71 eines Ausgangsübertragers 7, der eine galvanische Trennung des an die Sekundärwicklung 72 angeschlossenen Ausgangs 14 bewirkt. Ferner ist ein Blockkondensator 17 vorgesehen. Dieser dient dazu, die Abgabe von Gleichstromanteilen an das chirurgische Instrument 16 zu verhindern.

Das Tiefpassfilter 8 ist mit einer aktiven Dämpfung versehen. Sie umfasst eine Rückführung 9, an die ein Stromsensor 83 als Eingang angeschlossen ist. Der Stromsensor 83 ist in demselben Zweig wie die Kapazität 82 des Tiefpassfilters 8 angeordnet und bestimmt so den Stromfluss durch den die Kapazität 82. Durch die Bestimmung des Stroms kann ein entsprechendes Signal proportional zum gemessenen Strom rückgeführt werden über die Rückführung 9. In dieser ist eine Transferfunktion implementiert, die entsprechend dem gewünschten Verhalten des nun aktiv bedämpften Tiefpassfilter 8 gewählt ist. Im einfachsten Fall kann die Transferfunktion als ein Proportionalglied ausgeführt sein. Das Ausgangssignal der Rückführung 9 wird auf einen negativen Eingang eines Differenzglieds 91 aufgeschaltet zur Modifizierung des vom Steuersignalgenerator 40 erzeugten Referenzsignals, das an den positiven Eingang des Differenzglieds 91 angeschlossen ist. Das so modifizierte Referenzsignal wird am Ausgang des Differenzglieds 91 ausgegeben und ist angelegt an einen Eingang der Invertersteuerung 41 als Ansteuersignal für den Multilevel-Inverter 4. Auf diese Weise kann die Ausgangsspannung des Multilevel-Inverters 4 abhängig von der Rückführung 9 gesteuert werden. Somit können unerwünschte Resonanzen bereits im Ansatz verhindert werden. Ferner kann alternativ oder zusätzlich ein Stromsensor 84 vorgesehen sein, der an dem primärseitigen Anschluss des Ausgangsübertragers 7 oder in Serie zu dem Blockkondensator 17 angeordnet ist und so den Stromfluss durch den Ausgangsübertrager 7 bestimmt. Durch die Bestimmung des Stroms kann ein entsprechendes Signal proportional zum gemessenen Strom ebenfalls über die Rückführung 9 rückgeführt werden. In der Rückführung ist eine (entsprechend erweiterte) Transferfunktion implementiert, die entsprechend dem gewünschten, nun aktiv bedämpften Verhalten des durch die Induktivität 81 und den Blockkondensator 17 gebildeten LC-Filters gewählt ist.

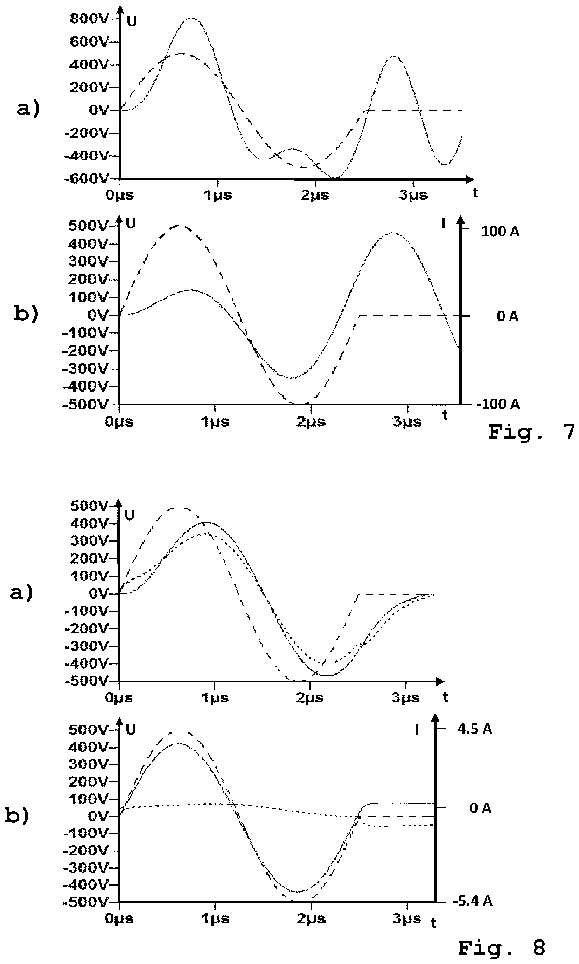

Die Wirkung der Rückführung 9 auf die Spannungs- bzw. Stromverläufe am Ausgang 14 ist in den Figuren 7a, b und Figur 8a, b dargestellt. In beiden Fällen ist vom Multilevel-Inverter 4 ein aus einer einzelnen Sinuswellenschwingung bestehendes pulsartiges Wechselspannungs-Signal erzeugt (wie dargestellt in Figur 9 e). Bei dem in Figur 7a dargestellten Fall ist die Last am Ausgang als hochohmig angenommen (im Bereich 100 kOhm). Dadurch ist der Ausgangsspannung (gestrichelte Linie) der von den Wechselrichter-Zellen 5 des Multilevel-Inverters 4 erzeugten Sinusschwingung zusätzlich eine Resonanzschwingung überlagert. Diese ergibt sich aus der Resonanzfrequenz des durch die Induktivitäten 81 und die Kapazität 82 gebildeten LC-Filters gemäß der bekannten Formel $f = \frac{1}{2 \cdot \pi \sqrt{L \cdot C}}$. Das sich so ergebende überlagerte Ausgangssignal ist mit der durchgezogenen Linie dargestellt. Man erkennt eine erhebliche Verformung der Kurve und ausgeprägtes Nachschwingen. - Der komplementäre Fall eines Kurzschluss ist in Figur 7b dargestellt. Man erkennt wieder die (identische) von den Wechselrichter-Zellen 5 des Multilevel-Inverters 4 erzeugte Sinuswellenschwingung (gestrichelte Linie). Hinzu tritt eine Überlagerung aus der Resonanzschwingung, die sich ergibt aus der Filter-Induktivität 81, die mit dem Blockkondensator 17 am Ausgang 14 resoniert. Der sich dabei ergebende Stromverlauf ist am Ausgang 14 mit der durchgezogenen Linie dargestellt. Man erkennt, dass sich eine erhebliche Störschwingung aufbaut.

In Figur 8a, b sind dieselben Fälle dargestellt, wobei das Filter 8 mittels der Rückführung 9 bedämpft ist. In Figur 8a ist wiederum der Fall mit hochohmiger Last gezeigt. Das ursprüngliche Ausgangssignal der Wechselrichter-Zellen 5 des Multilevel-Inverters 4 ist als Referenz weiterhin mit gestrichelter Linie dargestellt. Das mit einer Störung überlagerte tatsächliche Ausgangssignal ist über die Rückführung 9 unter Nutzung von Messsignalen des Stromsensors 83 rückgekoppelt und verändert durch den Eingriff an dem Differenzglied 91 das dem Multilevel-Inverter 4 zugeführte Referenzsignal. Dieses wird sozusagen gezielt verbogen, und es entsteht ein modifiziertes Referenzsignal, welches als Steuersignal tatsächlich an die Invertersteuerung 41 angelegt ist zur Ansteuerung der Wechselrichter-Zellen 5. Das so entstehende Ausgangssignal (s. gepunktete Linie, die einen geglätteten Verlauf darstellt) ist in der Weise gezielt "verbogen", dass der überlagerten Schwingung am Ausgang gezielt entgegen gewirkt wird. Das sich schließlich aus der gezielt "verbogen" erzeugten Spannung des Multilevel-Inverters 4 und der durch die Resonanzschwingung des Filters 8 ergebende tatsächliche Ausgangssignal ist mit der durchgezogenen Linie dargestellt. Man erkennt durch Vergleich mit Figur 7a ohne weiteres, dass das tatsächliche Ausgangssignal eine wesentlich harmonischere Sinusschwingung ist.

Entsprechendes gilt für den Kurzschlussfall unter Nutzung der Rückführung 9. Dieser Fall ist in Figur 8b dargestellt. Wieder ist das ursprüngliche Ansteuersignal, wie es als Referenzsignal von dem Steuersignalgenerator 40 erzeugt wird, mit gestrichelter Linie dargestellt. Das unter der Wirkung der Rückführung 9 unter Nutzung von Messsignalen des Stromsensors 84 schließlich erzeugte modifizierte Referenzsignal dient zur Ansteuerung der Wechselrichter-Zellen 5. Das so entstehende Ausgangssignal ist (nach Glättung) durch die gepunktete Linie dargestellt. Es fällt in Bezug auf die Spannungs-Amplitude ausgesprochen klein aus, was seinen Grund darin findet, dass die ungewollte Resonanzfrequenz recht dicht an der Frequenz der von dem Multilevel-Inverter 4 erzeugten Wechselspannung liegt. Damit wird nur ein sehr kleines tatsächliches Ansteuersignal für die Invertersteuerung 41 benötigt. Der sich dann ergebende tatsächliche Stromverlauf am Ausgang 14 ist wiederum mit der durchgezogenen Linie dargestellt. Man erkennt durch Vergleich mit Figur 7a ohne weiteres, dass das tatsächliche Ausgangssignal eine wesentlich harmonischere Sinusschwingung ist. Man erkennt deutlich durch Vergleich mit Figur 7b, dass die eigentliche Sinuswellenschwingung wesentlich genauer wiedergegeben ist (Zeitraum bis 2 ps) und danach parasitäres Nachschwingen wirksam unterdrückt ist (kein "Ringing"-Effekt). Die Rückkopplung unter Nutzung der Messsignale des Stromsensors 84 sorgt also für ein erheblich besseres und oberschwingungsärmeres sinusförmiges Ausgangssignal trotz des kritischen LC-Filters 8 mit dem Blockkondensator 17 am Ausgang 14.

Im Ergebnis können so mit dem Multilevel-Inverter 4 gemäß der Erfindung die auszugebenden Wechselspannung-Verläufe fein und präzise vorgegeben werden. Insbesondere besteht mit dem vom Referenzsignal angesteuerten Multilevel-Inverter 4 eine volle Kontrolle über die Kurvenform, und zwar insbesondere auch bei modulierten Ausgangssignalen. So können modulierte Ausgangssignale genau und reproduzierbar erzeugt werden, wie sie in den Figuren 9a bis 9e dargestellt sind. Um eine konstante Energieabgabe zu gewährleisten, ist es mit dem erfindungsgemäßen Multilevel-Inverter 4 weiterhin möglich, dass bei stärker modulierten Moden mit kürzeren Tastverhältnis die Höhe der abgegebenen Spannung so weit erhöht wird, dass trotz der kurzen Einschaltdauer die gleiche Energie an das elektrochirurgische Instrument 16 abgegeben wird wie bei den Moden mit längerer Einschaltdauer bzw. bei dem kontinuierlichen Modus.

Die Erfindung ermöglicht somit eine dynamischere und genauere Kontrolle über die abgegebene hochfrequente Wechselspannung, und zwar auch und gerade in gepulsten Moden. Dank der optionalen Rückkopplung können die Moden nochmals deutlich präziser eingehalten werden.

Ferner sei noch angemerkt, dass die Erfindung nicht auf Wechselrichter-Zellen 5 mit H-Brückenschaltung beschränkt ist. Es können auch andere Topologien für die Wechselrichter-Zellen 5 vorgesehen sein. Beispiele dafür zeigen die Figuren 6a und 6b, in denen alternative Topologien dargestellt sind, und zwar mit ebenfalls jeweils vier Schaltelementen 51' bis 54' bzw. 51" bis 54". So zeigt Figur 6a eine Ausführung der Wechselrichter-Zelle in der Bauart mit Neutralpunkt-Klemmung mittels Dioden 55, 56 und Figur 6b in der Bauart mit fliegendem Kondensator 57. Diese können, ähnlich zu den Wechselrichter-Zellen in H-Brückenschaltung, ebenfalls kaskadiert werden zur Erreichung von einer höheren Anzahl von Spannungsstufen.

In Fig. 10 ist ein alternatives Ausführungsbeispiel zu dem Ausführungsbeispiel gemäß Fig. 1 dargestellt. Gleiche oder gleichartige Elemente sind mit denselben Bezugsziffern bezeichnet. Es unterscheidet sich im Wesentlichen dadurch, dass das Tiefpassfilter 8 bei dem alternativen Ausführungsbeispiel zweistufig ausgeführt ist. Eine erste Stufe 8' des Tiefpassfilters ist weiterhin zur Glättung der erzeugten Wechselspannung unmittelbar am Ausgang des Multilevel-Inverters 4 angeordnet. Eine zweite Stufe 8" des Tiefpassfilter ist ausgangsseitig des Ausgangsübertragers 7 angeordnet. So erfolgt eine weitere Glättung noch kurz vor dem Ausgang, um insbesondere auch Störungen durch den Ausgangsübertrager 7 zu erfassen. Es sei angemerkt, dass auch die Streu-Induktivität des Ausgangsübertragers 7 zu der Induktivität 81 der zweiten Stufe 8" des Tiefpassfilters beitragen und diese ggf. zumindest zum Teil ersetzen kann.

Bei der Ausführungsform gemäß Fig. 10 sind doppelte Blockkondensatoren 17, 17' vorgesehen zur Erhöhung der Sicherheit. Es versteht sich, dass eine solche verdoppelte Anordnung auch bei den anderen Ausführungsbeispielen vorgesehen sein kann.

Am Beispiel von diesem Ausführungsbeispiel gemäß Fig. 10 ist auch eine zweckmäßige alternative Anordnung der Stromsensoren für die Rückführung dargestellt; diese kann auch bei den anderen Ausführungsbeispielen vorgesehen sein. Hierbei ist ein Stromsensor 18' in Reihe am Tiefpass 8 vorgesehen, genauer gesagt am Ausgang der ersten Stufe 8'. Als zweiter Stromsensor fungiert derjenige des kombinierten Strom- und Spannungssensors 18. Basierend auf diesen Signalen kann der tatsächliche abgegebene Strom gemessen werden (und wird über die Verarbeitungseinheit 19 an die Betriebssteuerung 10 übermittelt) wie auch der Stromfluss eingangsseitig des Ausgangsübertragers 7. Ferner ist ein Querstromdetektor gebildet. Er ist dazu ausgebildet, aus einer sich dabei ergebenden Stromdifferenz zu bestimmen, wie groß ein Strom durch eine Kapazität 82 des Tiefpass (hier der zweiten Stufe 8" des Tiefpass) ist. Dies kann von der Rückführung 9 erfasst und durch Veränderung der Ansteuerung des Multilevel-Inverters 4 kompensiert werden. Auf diese Weise können auch Stromverluste durch ansonsten nicht direkt messbare parasitäre Querkapazität, insbesondere des Ausgangsübertragers 7 bzw. von dem Tiefpass 8 mit seinen Stufen 8', 8", erfasst und ausgeglichen werden.

Ein weiteres Ausführungsbeispiel für einen Elektrochirurgie-Generator gemäß der vorliegenden Erfindung ist in Fig. 11 dargestellt. Er basiert auf dem in Fig. 1 dargestellten Ausführungsbeispiel, unterscheidet sich aber dadurch dass ein zweiter Ausgang 14* vorgesehen ist sowie eine Umschalteinrichtung 3. An ihren Eingang ist der Multilevel-Inverter 4 angeschlossen und an ihren einen Ausgang die Abgabeleitung 13, die über den Tiefpass 8 und Ausgangsübertrager 7 zum (ersten) Ausgang 14 für das elektrochirurgische Instrument 16 führt. An den anderen Ausgang der Umschalteinrichtung 3 ist über eine zweite Abgabeleitung 13*, einen zweiten Tiefpassfilter 8* und einen zweiten Ausgangsübertrager 7' ein zweiter Ausgang 14* angeschlossen. An diesen kann ein Anschlussstecker 15* für ein zweites Instrument (nicht dargestellt) angeschlossen sein, wobei es sich bei dem zweiten Instrument insbesondere um ein Ultraschallchirurgisches Instrument, wie bspw. ein Ultraschall-Skalpell, handeln kann. An den Ausgängen 14, 14* ist jeweils noch mindestens ein (nicht dargestellter) Blockkondensator 17 vorgesehen, wie bei dem in Fig. 1 gezeigten Ausführungsbeispiel.

Die Umschalteinrichtung 3 ist dazu ausgebildet, die von dem Multilevel-Inverter 4 erzeugte Wechselspannung wahlweise am Ausgang 14 an das dort angeschlossene Instrument 16, insbesondere das elektrochirurgische Instrument 16, oder am Ausgang 14* an das dort angeschlossene Instrument, insbesondere das ultraschallchirurgische Instrument, auszugeben. Somit kann mit demselben Elektrochirurgie-Generator 1 je nach Wunsch des Chirurgen ein elektrochirurgisches Instrument, wie bspw. ein Elektrokauter, oder ein ultraschallchirurgischen Instrument, wie bspw. eine Ultraschall-Dissektionsschere, eingesetzt werden. Der Wechsel zwischen den Instrumenten ist beträchtlich erleichtert und kann sogar intraoperativ erfolgen. Das Anwendungsfeld für den Elektrochirurgie-Generator ist somit deutlich verbreitert. Alternativ oder zusätzlich kann bei einer Variante wie in Fig. 12 dargestellt auch vorgesehen sein, dass die Wechselrichter-Zellen 5 schaltungsmäßig aufgeteilt sind. Dabei ist mindestens eine (aber nicht alle) der Wechselrichter-Zellen 5 mit dem zweiten Ausgang 14* verbunden und kann diesen bspw. mit einer Wechselspannung im Ultraschall-Frequenzbereich versorgen, während die übrigen Wechselrichter-Zellen 5-1 bis 5-4 weiterhin den Ausgang 14 mit hochfrequenter Wechselspannung versorgen. Auf diese Weise können auch zwei elektrochirurgische Instrumente parallel betrieben werden (auch in verschiedenen Modi), oder es kann ohne weiteres auch ein Instrument betrieben werden, welches sowohl Ultraschall als auch Hochfrequenzenergie nutzt.

**Patentansprüche**

1. Elektrochirurgie-Generator, der dazu ausgebildet ist eine hochfrequente Wechselspannung an ein elektrochirurgisches Instrument (16) abzugeben, umfassend eine Gleichspannungsversorgung (2) und einen Inverter für Hochspannung, der von der Gleichspannungsversorgung (2) gespeist ist und eine hochfrequente Wechselspannung mit variabler Spannung und Frequenz erzeugt, die an einem Ausgang (14) zum Anschluss des elektrochirurgischen Instruments (16) angelegt ist, **dadurch gekennzeichnet, dass** der Inverter als Multilevel-Inverter (4) ausgeführt ist und eine Mehrzahl von kaskadiert geschalteten Wechselrichter-Zellen (5) umfasst, die von einer Steuereinrichtung (41) angesteuert sind.

2. Elektrochirurgie-Generator nach Anspruch 1, **dadurch gekennzeichnet, dass** die Wechselrichter-Zellen (5), vorzugsweise ausgangsseitig, eine Potentialentkopplung aufweisen.

3. Elektrochirurgie-Generator nach dem vorangehenden Anspruch, **dadurch gekennzeichnet, dass** an dem Ausgang der jeweiligen Wechselrichter-Zelle (5) jeweils ein Übertrager (6) mit seiner Primärseite angeschlossen ist, wobei vorzugsweise die Sekundärseiten der Übertrager (6) verkettet sind zur Summierung der Sekundärspannungen der jeweiligen Übertrager (6), wobei weiter vorzugsweise die summierte Spannung über eine Abgableitung zum Ausgang (14) zum Anschluss des elektrochirurgischen Instruments (16) geführt ist.

4. Elektrochirurgie-Generator nach dem vorangehenden Anspruch, **dadurch gekennzeichnet, dass** die Übertrager (6) jeweils mit einem Transformator als Vorverstärker zum Hochsetzen der Spannung versehen sind, wobei vorzugsweise die Übertrager (6) mit dem jeweiligen Transformator baulich integriert ausgeführt sind.

5. Elektrochirurgie-Generator nach einem der vorangehenden Ansprüche, **dadurch gekennzeichnet, dass** die Wechselrichter-Zellen (5) gespeist sind von jeweils einer Gleichspannungsquelle (31), oder mehrere, mindestens zwei, Gruppen von Wechselrichter-Zellen (5) vorgesehen sind, wobei die Wechselrichter der jeweiligen Gruppe von einer Gleichspannungsquelle gemeinsam versorgt sind.

6. Elektrochirurgie-Generator nach einem der vorangehenden Ansprüche, **dadurch gekennzeichnet, dass** mehrere, mindestens zwei Gruppen (I, II) von Wechselrichter-Zellen (5) vorgesehen sind, wobei die Gruppen mit Gleichspannung in unterschiedlicher Höhe versorgt sind, wobei vorzugsweise eine Gruppe von den mehreren Gruppen mit einer mindestens doppelt so hohen, Gleichspannung versorgt ist als eine andere Gruppe von den mehreren Gruppen, wobei weiter vorzugsweise zur Versorgung mindestens einer der Gruppen mit unterschiedlicher Spannung jeweils mindestens ein insbesondere ratiometrischer, vorzugsweise bidirektional ausgebildeter, Gleichspannungswandler (42) vorgesehen ist.

7. Elektrochirurgie-Generator nach einem der vorangehenden Ansprüche, **dadurch gekennzeichnet, dass** Gleichspannungsquellen zur Versorgung der Wechselrichterzellen (5) galvanisch gekoppelt sind, und/oder die Gleichspannungsversorgung (2) als eine Festspannungsversorgung ausgebildet ist, insbesondere einen Gleichspannungszwischenkreis (20) mit einem festen Spannungsniveau umfasst, woran optionale Gleichspannungswandler (42) angeschlossen sind.

8. Elektrochirurgie-Generator nach einem der vorangehenden Ansprüche, **dadurch gekennzeichnet, dass** die Wechselrichter-Zellen (5) jeweils in der Bauart mit Neutralpunkt-Klemmung (55, 56) an ihrer Gleichspannungsversorgung oder mit fliegendem Kondensator (57) ausgeführt sind, oder die Wechselrichter-Zellen (5) in Reihe geschaltet sind und vorzugsweise jeweils in H-Brückenschaltung ausgeführt sind.

9. Elektrochirurgie-Generator nach einem der vorangehenden Ansprüche, **dadurch gekennzeichnet, dass** ein Steuersignalgenerator (40) für den Multilevel-Inverter (4) vorgesehen ist, der dazu ausgebildet ein Referenzsignal für die Ansteuerung des Multilevel-Inverters (4) zu erzeugen, wobei vorzugsweise das Referenzsignal ein Muster für von dem Elektrochirurgie-Generator (1) abzugebende Wechselspannung ist, insbesondere hinsichtlich Amplitude, Frequenz, Kurvenform und/oder Tastverhältnis, wobei weiter vorzugsweise die Kurvenform frei wählbar einstellbar ist.

10. Elektrochirurgie-Generator nach dem vorangehenden Anspruch, **dadurch gekennzeichnet, dass** der Steuersignalgenerator (40) eine Invertersteuerung (41) ansteuert, die dazu ausgebildet ist die Wechselrichter-Zellen (5) so anzusteuern, dass sie eine Ausgangsspannung gemäß dem Referenzsignal erzeugen.

11. Elektrochirurgie-Generator nach einem der vorangehenden Ansprüche, **dadurch gekennzeichnet, dass** die Wechselrichter-Zellen (5) mit einem Referenzsignal variabler Frequenz angesteuert werden.

12. Elektrochirurgie-Generator nach einem der vorangehenden Ansprüche, **dadurch gekennzeichnet, dass** an der Abgableitung (13) ein Ausgangsübertrager (7) als weitere galvanische Trenneinrichtung vorgesehen ist, die vorzugsweise mit einem Kondensator (17) am Ausgangsanschluss (14) als Gleichstromsperre zusammenwirkt, wobei der Ausgangsübertrager (7) vorzugsweise als Ausgangstransformator zur Haupt-Spannungsverstärkung ausgeführt ist.

13. Elektrochirurgie-Generator nach einem der vorangehenden Ansprüche, **dadurch gekennzeichnet, dass** in der Abgableitung (13) ein Tiefpassfilter (8) vorgesehen ist, das vorzugsweise als ein Filter mindestens zweiter Ordnung, insbesondere als ein LC-Filter, ausgeführt ist, wobei weiter vorzugsweise eine zweigeteilte Ausführung des Tiefpassfilters (8', 8") vorgesehen ist.

14. Elektrochirurgie-Generator nach dem vorangehenden Anspruch, **dadurch gekennzeichnet, dass** eine aktive Dämpfungseinrichtung für das Tiefpassfilter (8) vorgesehen ist, wobei vorzugsweise die aktive Dämpfungseinrichtung eine Rückführung umfasst, wobei die Rückführung (9) mindestens einen Stromsensor (83, 84, 18') am Tiefpassfilter (8) aufweist, und weiter vorzugsweise ein Querstromdetektor vorgesehen ist, durch den Stromfluss im Tiefpassfilter (8) und/oder Ausgangsübertrager (7) bestimmt werden kann.

15. Elektrochirurgie-Generator nach dem vorangehenden Anspruch, **dadurch gekennzeichnet, dass** ein Ausgangssignal der aktiven Dämpfungseinrichtung auf den Multilevel-Inverter (4) einwirkt, wobei vorzugsweise das Ausgangssignal der aktiven Dämpfungseinrichtung in eine Ansteuerung der Wechselrichter-Zellen (5) eingekoppelt ist.

16. Elektrochirurgie-Generator nach einem der vorangehenden Ansprüche, **dadurch gekennzeichnet, dass** mindestens ein weiterer Ausgang (14*) vorgesehen ist, an dem eine weitere von dem Multilevel-Inverter (4) erzeugte Wechselspannung angelegt ist, wobei vorzugsweise die mindestens eine weitere Wechselspannung eine niedrigere Frequenz als die hochfrequente Wechselspannung am Ausgang (14) für den Anschluss des elektrochirurgischen Instruments (16) aufweist, und insbesondere im Ultraschallbereich liegt.

17. Elektrochirurgie-Generator nach dem vorangehenden Anspruch, **dadurch gekennzeichnet, dass** mindestens eine Umschalteinrichtung (3) vorgesehen ist, welche dazu ausgebildet ist den Multilevel-Inverter (4) wahlweise mit einem der Ausgänge (14, 14*) zu verbinden.

18. Elektrochirurgie-Generator nach Anspruch 16 oder 17,
    **dadurch gekennzeichnet, dass** die Wechselrichter-Zellen (5) schaltungsmäßig so aufgeteilt sind, dass mindestens ein Teil der Wechselrichter-Zellen für einen Anschluss an dem mindestens einen weiteren Ausgang (14*) vorgesehen ist und ein anderer Teil der Wechselrichter-Zellen weiterhin den Ausgang (14) versorgt.

**Claims**

1. Electrosurgical generator that is designed to output a high-frequency AC voltage to an electrosurgical instrument (16), comprising a DC voltage supply (2) and a high-voltage inverter that is fed from the DC voltage supply (2) and generates a high-frequency AC voltage having a variable voltage and frequency that is applied to an output (14) for the connection of the electrosurgical instrument (16), **characterized in that** the inverter is configured as a multilevel inverter (4) and comprises a plurality of inverter cells (5) connected in a cascaded manner that are driven by a control device (41) .

2. Electrosurgical generator according to Claim 1, **characterized in that** the inverter cells (5) have potential decoupling, preferably at output.

3. Electrosurgical generator according to the preceding claim, **characterized in that** a respective transformer (6) is connected at the output of the respective inverter cell (5) with its primary side, wherein the secondary sides of the transformers (6) are preferably chained in order to sum the secondary voltages of the respective transformers (6), wherein the summed voltage is furthermore preferably channelled via an output line to the output (14) for the connection of the electrosurgical instrument (16).

4. Electrosurgical generator according to the preceding claim, **characterized in that** the transformers (6) are each provided with a transformer unit as preamplifier for stepping up the voltage, wherein the transformers (6) are preferably configured to be structurally integrated with the respective transformer unit.

5. Electrosurgical generator according to one of the preceding claims, **characterized in that** the inverter cells (5) are fed from in each case one DC voltage source (31) or a plurality of, at least two groups of inverter cells (5) are provided, wherein the inverters of the respective group are supplied jointly by one DC voltage source.

6. Electrosurgical generator according to one of the preceding claims, **characterized in that** a plurality of, at least two groups (I, II) of inverter cells (5) are provided, wherein the groups are supplied with DC voltage of different values, wherein preferably one group of the plurality of groups is supplied with a DC voltage that is at least twice as high as another group of the plurality of groups, wherein furthermore preferably provision is made for in each case at least one in particular ratiometric DC-to-DC converter (42), preferably of bidirectional design, for supplying at least one of the groups with a different voltage.

7. Electrosurgical generator according to one of the preceding claims, **characterized in that** DC voltage sources for supplying the inverter cells (5) are galvanically coupled, and/or the DC voltage supply (2) is designed as a fixed voltage supply, in particular comprises a DC link circuit (20) having a fixed voltage level, to which optional DC-to-DC converters (42) are connected.

8. Electrosurgical generator according to one of the preceding claims, **characterized in that** the inverter cells (5) are each configured with a type of structure with neutral point clamping (55, 56) at their DC voltage supply or with a floating capacitor (57), or the inverter cells (5) are connected in series and are preferably each configured in an H-bridge configuration.

9. Electrosurgical generator according to one of the preceding claims, **characterized in that** provision is made for a control signal generator (40) for the multilevel inverter (4) that is designed to generate a reference signal for driving the multilevel inverter (4), wherein the reference signal is preferably a pattern for AC voltage to be output by the electrosurgical generator (1), in particular with regard to amplitude, frequency, curve form and/or duty cycle, wherein the curve form is furthermore preferably able to be set freely as desired.

10. Electrosurgical generator according to the preceding claim, **characterized in that** the control signal generator (40) drives an inverter controller (41) that is designed to drive the inverter cells (5) such that they generate an output voltage in accordance with the reference signal.

11. Electrosurgical generator according to one of the preceding claims, **characterized in that** the inverter cells (5) are driven with a variable-frequency reference signal.

12. Electrosurgical generator according to one of the preceding claims, **characterized in that** provision is made for an output transformer (7) on the output line (13) as a further galvanic isolation device, which preferably interacts with a capacitor (17) on the output port (14) as DC current blocker, wherein the output transformer (7) is preferably configured as an output transformer unit for the main voltage amplification.

13. Electrosurgical generator according to one of the preceding claims, **characterized in that** provision is made, in the output line (13), for a low-pass filter (8), which is preferably configured as an at least second- order filter, in particular as an LC filter, wherein provision is furthermore preferably made for a two-part configuration of the low-pass filter (8' 8").

14. Electrosurgical generator according to the preceding claim, **characterized in that** provision is made for an active damping device for the low-pass filter (8), wherein the active damping device preferably comprises a feedback system, wherein the feedback system (9) has at least one current sensor (83, 84, 18') on the low-pass filter (8), and provision is furthermore preferably made for a transverse current detector, using which it is possible to determine current flow in the low-pass filter (8) and/or output transformer (7).

15. Electrosurgical generator according to the preceding claim, **characterized in that** an output signal from the active damping device acts on the multilevel inverter (4), wherein the output signal from the active damping device is preferably coupled into the driving of the inverter cells (5).

16. Electrosurgical generator according to one of the preceding claims, **characterized in that** provision is made for at least one further output (14*) to which a further AC voltage generated by the multilevel inverter (4) is applied, wherein the at least one further AC voltage preferably has a lower frequency than the high-frequency AC voltage at the output (14) for the connection of the electrosurgical instrument (16), and is in particular in the ultrasonic range.

17. Electrosurgical generator according to the preceding claim, **characterized in that** provision is made for at least one changeover device (3) that is designed to selectively connect the multilevel inverter (4) to one of the outputs (14, 14*).

18. Electrosurgical generator according to Claim 16 or 17, **characterized in that** the inverter cells (5) are divided in terms of circuitry such that at least one portion of the inverter cells is provided for connection to the at least one further output (14*) and another portion of the inverter cells furthermore supplies the output (14).

**Revendications**

1. Générateur pour électrochirurgie, lequel est configuré pour délivrer une tension alternative à haute fréquence à un instrument pour électrochirurgie (16), comprenant une alimentation en tension continue (2) et un onduleur pour la haute tension, lequel est alimenté par l'alimentation en tension continue (2) et génère une tension alternative à haute fréquence ayant une tension et une fréquence variables, laquelle est appliquée à une sortie (14) destinée au raccordement de l'instrument pour électrochirurgie (16), **caractérisé en ce que** l'onduleur est réalisé sous la forme d'un onduleur à niveaux multiples (4) et comporte une pluralité de cellules de convertisseur statique (5) branchées en cascade, lesquelles sont commandées par un dispositif de commande (41).

2. Générateur pour électrochirurgie selon la revendication 1, **caractérisé en ce que** les cellules de convertisseur statique (5) présentent un découplage de potentiel, de préférence du côté de la sortie.

3. Générateur pour électrochirurgie selon la revendication précédente, **caractérisé en ce qu'**un transmetteur (6) est respectivement raccordé par son côté primaire à la sortie de la cellule de convertisseur statique (5) respective, les côtés secondaires des transmetteurs (6) étant de préférence chaînés en vue d'additionner les tensions secondaires des transmetteurs (6) respectifs, la tension totalisée étant encore de préférence acheminée par le biais d'une ligne de distribution à la sortie (14) destinée au raccordement de l'instrument pour électrochirurgie (16).

4. Générateur pour électrochirurgie selon la revendication précédente, **caractérisé en ce que** les transmetteurs (6) sont respectivement pourvus d'un transformateur faisant office de préamplificateur servant à élever la tension, les transmetteurs (6) étant de préférence réalisés avec le transformateur respectif intégré par construction.

5. Générateur pour électrochirurgie selon l'une des revendications précédentes, **caractérisé en ce que** les cellules de convertisseur statique (5) sont alimentées respectivement par une source de tension continue (31), ou plusieurs, au moins deux, groupes de cellules de convertisseur statique (5) sont présents, les convertisseurs statiques du groupe respectif étant alimentés en commun par une source de tension continue.

6. Générateur pour électrochirurgie selon l'une des revendications précédentes, **caractérisé en ce que** plusieurs, au moins deux groupes (I, II) de cellules de convertisseur statique (5) sont présents, les groupes étant alimentés avec une tension continue à un niveau différent, un groupe des plusieurs groupes étant de préférence alimenté avec une tension continue à un niveau au moins deux fois plus élevé qu'un autre groupe des plusieurs groupes, encore de préférence, au moins un convertisseur de tension continue (42), de préférence de configuration bidirectionnelle, notamment ratiométrique, étant encore de préférence présent pour l'alimentation d'au moins l'un des groupes avec une tension différente.

7. Générateur pour électrochirurgie selon l'une des revendications précédentes, **caractérisé en ce que** des sources de tension continue destinées à l'alimentation des cellules de convertisseur statique (5) sont couplées galvaniquement et/ou la source de tension continue (2) est réalisée sous la forme d'une alimentation en tension fixe, notamment un circuit intermédiaire de tension continue (20) avec un niveau de tension fixé auquel sont raccordés des convertisseurs de tension (42) optionnels.

8. Générateur pour électrochirurgie selon l'une des revendications précédentes, **caractérisé en ce que** les cellules de convertisseur statique (5) sont respectivement réalisées selon le modèle clampé par le neutre (55, 56) au niveau de leur alimentation en tension continue ou avec condensateur flottant (57), ou alors les cellules de convertisseur statique (5) sont branchées en série et sont de préférence respectivement réalisées en circuit de pont en H.

9. Générateur pour électrochirurgie selon l'une des revendications précédentes, **caractérisé en ce qu'**un générateur de signal de commande (40) est présent pour l'onduleur à niveaux multiples (4), lequel est configuré pour générer un signal de référence pour la commande de l'onduleur à niveaux multiples (4), le signal de référence étant de préférence un modèle pour une tension alternative à délivrer par le générateur pour électrochirurgie (1), notamment pour ce qui concerne l'amplitude, la fréquence, la forme de courbe et/ou le rapport cyclique, la forme de courbe étant encore de préférence réglable avec sélection libre.

10. Générateur pour électrochirurgie selon la revendication précédente, **caractérisé en ce que** le générateur de signal de commande (40) commande un circuit onduleur (41), lequel est configuré pour commander les cellules de convertisseur statique (5) de telle sorte qu'elles génèrent une tension de sortie conformément au signal de référence.

11. Générateur pour électrochirurgie selon l'une des revendications précédentes, **caractérisé en ce que** les cellules de convertisseur statique (5) sont commandées avec un signal de référence à fréquence variable.

12. Générateur pour électrochirurgie selon l'une des revendications précédentes, **caractérisé en ce qu'**un transmetteur de sortie (7) est présent au niveau de la ligne de distribution (13) en tant que dispositif de séparation galvanique supplémentaire, lequel coopère de préférence avec un condensateur (17) au niveau de la borne de sortie (14) en tant que blocage du courant continu, le transmetteur de sortie (7) étant de préférence réalisé sous la forme d'un transformateur de sortie destiné à l'amplification de tension principale.

13. Générateur pour électrochirurgie selon l'une des revendications précédentes, **caractérisé en ce qu'**un filtre passe-bas (8) est présent dans la ligne de distribution (13), lequel est de préférence réalisé sous la forme d'un filtre au moins du deuxième ordre, notamment sous la forme d'un filtre LC, une exécution en deux parties du filtre passe-bas (8', 8") étant encore de préférence présente.

14. Générateur pour électrochirurgie selon la revendication précédente, **caractérisé en ce qu'**un dispositif d'amortissement actif est présent pour le filtre passe-bas (8), le dispositif d'amortissement actif comportant de préférence une contre-réaction, la contre-réaction (9) possédant au moins un capteur de courant (83, 84, 18') au niveau du filtre passe-bas (8), et encore de préférence un détecteur de courant transversal étant présent, par lequel le flux de courant dans le filtre passe-bas (8) et/ou le transmetteur de sortie (7) peut être déterminé.

15. Générateur pour électrochirurgie selon la revendication précédente, **caractérisé en ce qu'**un signal de sortie du dispositif d'amortissement actif agit sur l'onduleur à niveaux multiples (4), le signal de sortie du dispositif d'amortissement actif étant de préférence injecté dans une commande des cellules de convertisseur statique (5).

16. Générateur pour électrochirurgie selon l'une des revendications précédentes, **caractérisé en ce qu'**au moins une sortie supplémentaire (14*) est présente, à laquelle est appliquée une tension alternative supplémentaire générée par l'onduleur à niveaux multiples (4), l'au moins une tension alternative supplémentaire présentant une fréquence inférieure à la tension alternative à haute fréquence à la sortie (14) pour le raccordement de l'instrument pour électrochirurgie (16), et qui se trouve notamment dans la plage des ultrasons.

17. Générateur pour électrochirurgie selon la revendication précédente, **caractérisé en ce qu'**au moins un dispositif d'inversion (3) est présent, lequel est configuré pour relier l'onduleur à niveaux multiples (4) sélectivement à l'une des sorties (14, 14*).

18. Générateur pour électrochirurgie selon la revendication 16 ou 17, **caractérisé en ce que** les cellules de convertisseur statique (5) sont réparties en terme de circuit de telle sorte qu'au moins une partie des cellules de convertisseur statique est prévue pour un raccordement à l'au moins une sortie supplémentaire (14*) et une autre partie des cellules de convertisseur statique alimente en outre la sortie (14).

Fig. 1

a)          b)

Fig. 2

**Fig. 3**

**Fig. 4**

Fig. 5

a)                                    b)

Fig. 6

Fig. 7

Fig. 8

Fig. 9

Fig. 10

Fig. 11

**Fig. 12**

**Fig. 13**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2514380 B1